(19) Europäisches Patentamt / European Patent Office / Office européen des brevets

(11) **EP 4 778 504 A1**

(12) **EUROPEAN PATENT APPLICATION**
published in accordance with Art. 153(4) EPC

(43) Date of publication:
**22.07.2026   Bulletin 2026/30**

(21) Application number: **24897199.6**

(22) Date of filing: **31.10.2024**

(51) International Patent Classification (IPC):
*A61K 6/30* (2020.01)        *A61K 6/15* (2020.01)
*A61K 6/17* (2020.01)        *A61K 6/70* (2020.01)
*A61K 6/831* (2020.01)       *A61K 6/842* (2020.01)
*A61K 6/878* (2020.01)       *C01B 33/12* (2006.01)

(52) Cooperative Patent Classification (CPC):
A61K 6/15; A61K 6/17; A61K 6/30; A61K 6/70;
A61K 6/831; A61K 6/842; A61K 6/878; C01B 33/12

(86) International application number:
**PCT/JP2024/038942**

(87) International publication number:
**WO 2025/115510 (05.06.2025 Gazette 2025/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **29.11.2023   JP 2023201789
29.11.2023   JP 2023201788**

(71) Applicant: **Tokuyama Dental Corporation
Tokyo 110-0016 (JP)**

(72) Inventors:
• **FUJIMORI, Yuji**
  **Tokyo 110-0016 (JP)**
• **HASHIMOTO, Akari**
  **Tokyo 110-0016 (JP)**
• **KIRA, Ryuta**
  **Tokyo 110-0016 (JP)**

(74) Representative: **Hoffmann Eitle
Patent- und Rechtsanwälte PartmbB
Arabellastraße 30
81925 München (DE)**

(54) **DENTAL-USE CURABLE COMPOSITION, INORGANIC POWDER GRANULAR BODY, AND METHOD FOR PRODUCING SAME**

(57)   The present invention relates to a dental curable composition containing a polymerizable monomer (M), and a specific inorganic particle powder (C) composed of aggregated particles (c) consisting of inorganic particles (a) that constitute an inorganic particle powder (A) having a specific average particle diameter and zeta potential, and inorganic particles (b) that constitute an inorganic particle powder (B) having a specific average particle diameter and zeta potential, and further containing at least one selected from the inorganic particle powder (A) and a specific organic-inorganic composite particle powder (D), with the content of each particle powder in a specific range. According to the present invention, it is possible to provide a dental curable composition that exhibits high dispersibility of an inorganic particle powder in the composition, good discharging properties from a syringe, appropriate fluidity, little change in fluidity over time, and high mechanical strength after curing.

Fig. 1

**Description**

Technical Field

The present invention relates to a dental curable composition, an inorganic particle powder, and a method for producing the same.

Background Art

A dental curable composition contains, as main components, a polymerizable monomer (monomer), an inorganic filler (filler) composed of an assembly of inorganic particles (inorganic particle powder), and a polymerization initiator, and among them, a composite resin is one of the materials most frequently used in dental treatment as a material for repairing a tooth defect or a cavity after removing dental caries.

A composite resin is required to be excellent in various physical properties such as operability in a paste state before polymerization and curing, and aesthetics and mechanical strength of a cured product obtained after polymerization and curing.

In recent years, a flowable composite resin has been developed, which is designed to be able to directly fill a cavity with paste from the tip of a needle called a needle tip, by mounting a needle having a small hole on a syringe containing a composite resin paste, and is used more frequently in clinical sites because it allows for easier restoration of teeth.

The flowable composite resin is required to have good operability in filling the flowable composite resin into a cavity or the like, specifically, good discharging properties when discharged from a syringe equipped with a needle tip, and to have appropriate fluidity in a paste state depending on the case or the like. For example, when the fluidity is too high in the paste state, the paste tends to sag, resulting in poor shapability (property of being able to maintain its shape without deformation due to natural flow when left to stand), so it is necessary for the paste to have appropriate fluidity.

Flowable composite resins have improved discharging properties from the syringe by relatively lowering the content of inorganic particle powder to increase fluidity; however, the increased fluidity can sometimes make it difficult to achieve good shapability.

To address these issues, PTL 1 discloses a technique that improves the various physical properties of a composite resin by using together two types of inorganic particle powder with opposite zeta potential polarities (in water) and specific average particle diameters and specific surface areas in each specific ratio. In Examples, it is described that a dental curable composition containing a polymerizable monomer, a silica-based composite oxide with an average particle diameter of 50 nm to 1 $\mu$m, and a crystalline rare earth metal fluoride with a specific surface area of 25 to 100 $m^2$/g exhibits good discharging properties (having a consistency suitable for discharging from a syringe) and moderate fluidity that suppresses paste sagging.

Citation List

Patent Literature

PTL 1: WO 2023/085201 A

Summary of Invention

Technical Problem

As described above, PTL 1 discloses that a dental curable composition (paste-like) with appropriate fluidity can be obtained by using together a silica-based composite oxide and a crystalline rare earth metal fluoride with opposite zeta potential polarities.

However, PTL 1 does not discuss the change in fluidity of the dental curable composition over time. According to the inventors' research, it was found that the dental curable composition described in PTL 1 may exhibit increased fluidity and lose its appropriate fluidity after being stored for a long period (for example, about one week at 50°C).

Therefore, an object of the present invention is to provide a dental curable composition that exhibits high dispersibility of an inorganic particle powder in the composition, good discharging properties from a syringe, appropriate fluidity, little change in fluidity over time, and high mechanical strength after curing.

Another object of the present invention is to provide an inorganic particle powder with good dispersibility when blended into a dental curable composition, and a method for producing the inorganic particle powder.

Solution to Problem

The inventors conducted extensive research to achieve the above objectives. As a result, they have found that the above problems can be solved by a dental curable composition containing a polymerizable monomer (M), and a specific inorganic particle powder (C) composed of aggregated particles (c) consisting of inorganic particles (a) that constitute an inorganic particle powder (A) having a specific average particle diameter and zeta potential, and inorganic particles (b) that constitute an inorganic particle powder (B) having a specific average particle diameter and zeta potential, and further containing at least one selected from the inorganic particle powder (A) and a specific organic-inorganic composite particle powder (D), with the content of each particle powder in a specific range, and have completed the present invention.

The gist of the present invention is as follows [1] to [12].

[1] A dental curable composition including a composition containing

100 parts by mass of a polymerizable monomer (M), and
10 to 100 parts by mass of an inorganic particle powder (C),
the inorganic particle powder (C) composed of aggregated particles (c) consisting of inorganic particles (a) that constitute an inorganic particle powder (A) having an average primary particle diameter of 50 nm to 1 $\mu$m as measured by electron microscopy, the inorganic particle powder (A) composed of a plurality of inorganic particles (a) composed of the same type of material, exhibiting a negative zeta potential polarity when measured in water, and inorganic particles (b) that constitute an inorganic particle powder (B) having an average primary particle diameter of 1 to 300 nm as measured by electron microscopy, the inorganic particle powder (B) composed of a plurality of inorganic particles (b) composed of the same type of material, exhibiting a positive zeta potential polarity when measured in water,
an average value of a total mass of the inorganic particles (b) relative to a total mass of 100 parts by mass of the inorganic particles (a) contained in each of the aggregated particles (c) that constitute the inorganic particle powder (C) being within a range of 20 to 300 parts by mass,
the inorganic particle powder (C) having an average aggregate particle diameter of 1 to 50 $\mu$m, defined as a median diameter in a volume-based particle size distribution measured by a laser diffraction-scattering method,
in which the dental curable composition further contains at least one of the inorganic particle powder (A) and an organic-inorganic composite particle powder (D),
the organic-inorganic composite particle powder (D) is composed of organic-inorganic composite particles (d) consisting of a composite material, which is a composite material of the inorganic particle powder (A) and a resin, and in which the inorganic particle powder (A) has a content of 60 to 90% by mass in the composite material, and the organic-inorganic composite particle powder (D) has an average particle diameter of 1 to 100 $\mu$m, defined as a median diameter in the volume-based particle size distribution measured by a laser diffraction-scattering method,
the inorganic particle powder (A) has a total content of, regardless of its form of inclusion, 170 to 270 parts by mass, and the inorganic particle powder (B) has a total content of, regardless of its form of inclusion, 5 to 50 parts by mass in the composition.

[2] The dental curable composition according to [1], in which, in each aggregated particle (c) constituting the inorganic particle powder (C), the inorganic particles (b) are uniformly dispersed in the inorganic particles (a).

[3] The dental curable composition according to [1] or [2], in which the inorganic particles (a) consist of a silica-based inorganic compound, and the inorganic particles (b) consist of a crystalline rare earth metal fluoride.

[4] The dental curable composition according to [1] to [3], in which a full width at half maximum of a maximum intensity peak derived from the crystalline rare earth metal fluoride in an X-ray diffraction pattern obtained when X-ray diffraction measurement is performed on the inorganic particle powder (B) is 0.3° or more.

[5] The dental curable composition according to [1] to [4], in which the inorganic particle powder (C) is obtained by spray drying a homogeneous mixed slurry obtained by mixing a slurry (Sa) in which 100 parts by mass of the inorganic particle powder (A) are dispersed in a dispersion medium, and a slurry (Sb) in which 20 to 300 parts by mass of the inorganic particle powder (B) are dispersed in a dispersion medium.

[6] A method for producing the dental curable composition according to any one of [1] to [5], including mixing the polymerizable monomer (M), the inorganic particle powder (C), and at least one of the inorganic particle powder (A) and the organic-inorganic composite particle powder (D).

[7] The method for producing the dental curable composition according to [6], in which the inorganic particle powder (C) is produced by spray drying a homogeneous mixed slurry obtained by mixing a slurry (Sa) in which 100 parts by mass of the inorganic particle powder (A) are dispersed in a dispersion medium, and a slurry (Sb) in which 20 to 300 parts by mass of the inorganic particle powder (B) are dispersed in a dispersion medium.

[8] The method for producing the dental curable composition according to [7], wherein the mixed slurry does not contain an ionic surfactant.

[9] The method for producing the dental curable composition according to [7] or [8], wherein the mixed slurry has a concentration of less than 40% by mass.

[10] An inorganic particle powder, which is an inorganic particle powder (C) composed of aggregated particles (c) consisting of inorganic particles (a) that constitute an inorganic particle powder (A) having an average primary particle diameter of 50 nm to 1 $\mu$m as measured by electron microscopy, the inorganic particle powder (A) composed of a plurality of inorganic particles (a) composed of the same type of material, exhibiting a negative zeta potential polarity when measured in water, and inorganic particles (b) that constitute an inorganic particle powder (B) having an average primary particle diameter of 1 to 300 nm as measured by electron microscopy, the inorganic particle powder (B) composed of a plurality of inorganic particles (b) composed of the same type of material, exhibiting a positive zeta potential polarity when measured in water,

an average value of a total mass of the inorganic particles (b) relative to a total mass of 100 parts by mass of the inorganic particles (a) contained in each of the aggregated particles (c) that constitute the inorganic particle powder (C) being within a range of 20 to 300 parts by mass,

the inorganic particle powder having an average aggregate particle diameter of 1 to 50 $\mu$m, defined as a median diameter in a volume-based particle size distribution measured by a laser diffraction-scattering method, and being free of an ionic surfactant.

[11] A method for producing the inorganic particle powder according to [10], including a step of spray drying a homogeneous mixed slurry obtained by mixing a slurry (Sa) in which 100 parts by mass of the inorganic particle powder (A) are dispersed in a dispersion medium, and a slurry (Sb) in which 20 to 300 parts by mass of the inorganic particle powder (B) are dispersed in a dispersion medium, in which the mixed slurry does not contain an ionic surfactant.

[12] The method for producing the inorganic particle powder according to [11], wherein the mixed slurry has a concentration of less than 40% by mass.

Advantageous Effects of Invention

According to the present invention, it is possible to provide a dental curable composition that exhibits high dispersibility of an inorganic particle powder in the composition, good discharging properties from a syringe, appropriate fluidity, little change in fluidity over time, and high mechanical strength after curing.

Furthermore, according to the present invention, it is possible to provide an inorganic particle powder with good dispersibility when blended into a dental curable composition, and a method for producing the inorganic particle powder.

Brief Description of Drawings

[Fig. 1] Fig. 1 is a diagram schematically showing inorganic particle powder (A) to (C) in the present invention.

[Fig. 2] Fig. 2 is an image of a cured product of a dental curable composition of Example 1 observed with a scanning electron microscope.

[Fig. 3] Fig. 3 is an image of a cured product of a dental curable composition of Comparative Example 8 observed with a scanning electron microscope.

Description of Embodiments

[Dental Curable Composition]

The dental curable composition of the present invention contains a polymerizable monomer (M) and an inorganic particle powder (C) composed of aggregated particles (c). The dental curable composition of the present invention further contains at least one of an inorganic particle powder (A) and an organic-inorganic composite particle powder (D) as a particle powder different from the inorganic particle powder (C). Here, since the aggregated particles (c) are aggregated particles consisting of inorganic particles (a) constituting the inorganic particle powder (A) and inorganic particles (b) constituting the inorganic particle powder (B), the dental curable composition of the present invention may contain the inorganic particle powder (A) and the inorganic particle powder (B) in specific forms, specifically, (A) in the form of (C), and (A) in its original form or in the form of (D) while (B) in the form of (C).

Hereinafter, the components of the dental curable composition of the present invention will be described in detail.

In the description herein, unless otherwise specified, the expression "x to y" using numerical values x and y means "x or more and y or less". In the event that only the numerical value y is indicated with a unit in such representation, such unit shall also apply to the numerical value x. Further, in the description herein, the term "(meth)acrylic" means both "acrylic" and "methacrylic". Similarly, the term "(meth)acrylate" means both "acrylate" and "methacrylate", and the term "(meth)acryloyl" means both "acryloyl" and "methacryloyl".

<Polymerizable monomer (M)>

The dental curable composition of the present invention contains a polymerizable monomer. As the polymerizable monomer, polymerizable monomers used in conventional dental curable compositions, such as radically polymerizable monomers and cationically polymerizable monomers, can be used without particular limitation. Among these, it is preferable to use a (meth)acrylate-based polymerizable monomer that is generally used, specifically, an acidic group-containing (meth)acrylate-based polymerizable monomer, a hydroxy group-containing (meth)acrylate-based polymerizable monomer, or a monofunctional or polyfunctional (meth)acrylate-based polymerizable monomer having no such substituents.

Examples of the (meth)acrylate-based polymerizable monomer that can be suitably used include the followings. That is, examples of the acidic group-containing (meth)acrylate-based polymerizable monomer include (meth)acrylic acid, N-(meth)acryloyl-p-aminobenzoic acid, 2-(meth)acryloyloxybenzoic acid, 2-(meth)acryloyloxyethyl phenyl hydrogen phosphate, and 2-(meth)acryloyloxyethyl phosphonic acid. Examples of the hydroxy group-containing (meth)acrylate-based polymerizable monomer include 2-hydroxyethyl (meth)acrylate, 3-hydroxypropyl (meth)acrylate, 4-hydroxybutyl (meth)acrylate, 6-hydroxyhexyl (meth)acrylate, 2,2-bis[(3-methacryloyloxy-2-hydroxypropyloxy)phenyl]propane, and 2,2-bis[4-(4-methacryloyloxy)-3-hydroxybutoxyphenyl]propane. Furthermore, examples of the monofunctional or polyfunctional (meth)acrylate-based polymerizable monomer having no such substituents include methyl (meth)acrylate, ethyl (meth)acrylate, triethylene glycol dimethacrylate, tetraethylene glycol dimethacrylate, neopentyl glycol dimethacrylate, 1,6-hexanediol dimethacrylate, 1,9-nonanediol dimethacrylate, 1,6-bis(methacrylethyloxycarbonylamino)trimethylhexane, and 2,2-bis(4-methacryloyloxypolyethoxyphenyl)propane.

Among these polymerizable monomers, bifunctional or higher functional, more preferably bifunctional to tetrafunctional polymerizable monomers are preferable because of high polymerizability, and particularly high mechanical strength of the cured product. These polymerizable monomers may be used singly or as a mixture of different kinds thereof.

<Inorganic particle powder (C)>

The inorganic particle powder (C) contained in the dental curable composition of the present invention is composed of aggregated particles (c) consisting of inorganic particles (a) that constitute an inorganic particle powder (A) having an average primary particle diameter of 50 nm to 1 $\mu$m as measured by electron microscopy, the inorganic particle powder (A) composed of a plurality of inorganic particles (a) composed of the same type of material, exhibiting a negative zeta potential polarity when measured in water, and inorganic particles (b) that constitute an inorganic particle powder (B) having an average primary particle diameter of 1 to 300 nm as measured by electron microscopy, the inorganic particle powder (B) composed of a plurality of inorganic particles (b) composed of the same type of material, exhibiting a positive zeta potential polarity when measured in water.

The average value of the total mass of the inorganic particles (b) relative to a total mass of 100 parts by mass of the inorganic particles (a) contained in each of the aggregated particles (c) that constitute the inorganic particle powder (C) is within the range of 20 to 300 parts by mass, and the inorganic particle powder (C) has an average aggregate particle diameter of 1 to 50 $\mu$m, defined as a median diameter in a volume-based particle size distribution measured by a laser diffraction-scattering method.

Hereinafter, the components constituting the inorganic particle powder (C) will be described in detail.

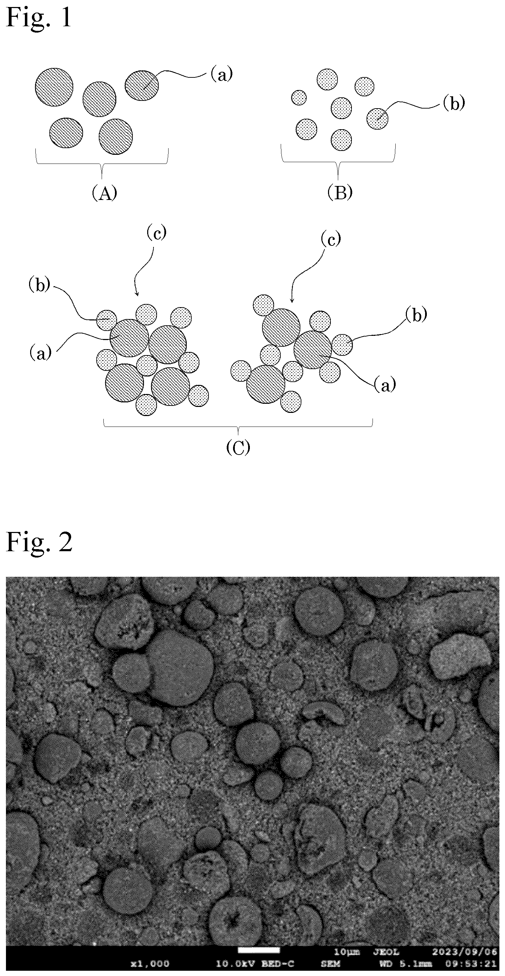

The inorganic particle powder (A), which is contained as the inorganic particle powder (C) in the dental curable composition of the present invention, is composed of a plurality of the inorganic particles (a), as schematically shown in Fig. 1. The inorganic particle powder (A) is an assembly of the inorganic particles (a), which are primary particles.

The inorganic particle powder (A), composed of a plurality of the inorganic particles (a), exhibits a negative zeta potential polarity when measured in water, and the plurality of inorganic particles (a) are composed of the same type of material. "Composed of the same type of material" means that the plurality of inorganic particles (a) constituting the inorganic particle powder (A) have the same chemical structure with one another.

The inorganic particle powder (B), which is included as the inorganic particle powder (C) in the dental curable composition of the present invention, is composed of a plurality of the inorganic particles (b), as schematically shown in Fig. 1. The inorganic particle powder (B) is an assembly of the inorganic particles (b), which are primary particles.

The inorganic particle powder (B), composed of a plurality of the inorganic particles (b), exhibits a positive zeta potential polarity when measured in water, and the inorganic particles (b) are composed of the same type of material. "Composed of the same type of material" means that the plurality of inorganic particles (b) constituting the inorganic particle powder (B) have the same chemical structure with one another.

Here, the polarity of the zeta potential measured in water refers to the polarity (sign, i.e., positive or negative) of the zeta potential measured by an electrophoretic light scattering method for the inorganic particle powder dispersed in ion-exchanged water at pH 7.

As schematically shown in Fig. 1, the inorganic particle powder (C) is composed of the aggregated particles (c) consisting of the inorganic particles (a) that constitute the inorganic particle powder (A) and the inorganic particles (b) that constitute the inorganic particle powder (B). The aggregated particles (c) are composed of a plurality of the inorganic particles (a) and a plurality of the inorganic particles (b), and any inorganic particle constituting the aggregated particle is in contact with at least one other inorganic particle constituting the aggregated particle. The formation of the aggregated particles (c) can be confirmed by electron microscopy.

The inorganic particle powder (C) is an assembly of a plurality of the aggregated particles (c).

When the inorganic particle powder (C) is blended into a dental curable composition, the generation of aggregates, particularly aggregates caused by the inorganic particles (b), is suppressed. For example, when crystalline rare earth metal fluoride is used as the inorganic particles (b), aggregates of crystalline rare earth metal fluoride generally tend to occur; however, when blended into a dental curable composition as the inorganic particle powder (C), the generation of aggregates is suppressed. Therefore, the dental curable composition of the present invention has good dispersibility of the inorganic particle powder, and the change in fluidity over time can be reduced.

The reason thereof is not certain, but it is presumed to be as follows. That is, since the inorganic particle powder (C) is composed of the aggregated particles (c), and the inorganic particles (b) of crystalline rare earth metal fluoride or the like and the inorganic particles (a) of a silica-based inorganic compound or the like are dispersed and mixed at the primary particle level, the formation of aggregates of the inorganic particles (b) of crystalline rare earth metal fluoride or the like is suppressed, resulting in good dispersibility. Furthermore, it is believed that by suppressing the formation of aggregates of the inorganic particles (b) of crystalline rare earth metal fluoride or the like, the change in fluidity of the dental curable composition over time can be reduced.

The inorganic particle powder (C) can be produced, for example, by spray drying a mixed slurry containing the inorganic particle powder (A) and the inorganic particle powder (B), as to be described later. With the inorganic particle powder (C), an increase in viscosity of the slurry used during producing can be suppressed, making it easier to produce by spray drying.

The reason thereof is not entirely clear, but it is presumed to be as follows.

That is, as described above, the inorganic particles (a) exhibit a negative zeta potential in water, while the inorganic particles (b) exhibit a positive zeta potential in water. In the present invention, the average primary particle diameter of each of the inorganic particle powder (A) composed of the inorganic particles (a) and the inorganic particle powder (B) composed of the inorganic particles (b) are set within a specific range, and the content of the inorganic particles (b) relative to the inorganic particles (a) is also set within a specific range. Therefore, in the slurry used for spray drying, units are formed in which the inorganic particles (a) exhibiting a negative zeta potential are surrounded by the inorganic particles (b) exhibiting a positive zeta potential. It is presumed that since these units repel each other due to the positive charge, the increase in viscosity is suppressed.

Hereinafter, the structure of the inorganic particle powder (C) will be described in more detail. First, the inorganic particle powder (A) and the inorganic particle powder (B), which are part of the inorganic particle powder (C) and are used as raw materials for producing the inorganic particle powder (C), will be described.

The inorganic particle powder (A) in the present invention is composed of a plurality of the inorganic particles (a).

The average primary particle diameter of the inorganic particle powder (A) is 50 nm to 1 $\mu$m. The average primary particle diameter is measured as follows using a scanning electron microscope or a transmission electron microscope. That is, the circular equivalent diameter $X_i$ (diameter of a circle having the same area as the area of a target particle) of each inorganic particle (a) is measured by performing image analysis on 30 or more, preferably 100 or more of n inorganic primary particles, where the inorganic particles (a) measured are randomly selected from a captured image by an electron microscope in which light and darkness are clear and the outline of the particle can be discriminated, and the average primary particle diameter means the average particle (volume) diameter: X, which is calculated by the formula: $X = \{(\Sigma X_i^3)/n\}^{1/3}$ based on the sum of $X_i^3$ from the first to the n-th particle: $\Sigma X_i^3$.

By setting the average primary particle diameter of the inorganic particle powder (A) to 50 nm to 1 $\mu$m, and by setting the average primary particle diameter of the inorganic particle powder (B) and the mass ratio of the inorganic particles (a) and the inorganic particles (b) contained in the aggregated particles (c) within a specific range as to be described later, it becomes easier to adjust the viscosity of the slurry during the production of the inorganic particle powder (C) to a lower level. Therefore, when the average primary particle diameter of the inorganic particle powder (A) is 50 nm to 1 $\mu$m, it becomes easier to obtain the inorganic particle powder (C) by the spray drying method. From these viewpoints, and from the viewpoint of improving the polishability of the cured product when blended into the dental curable composition, the average primary particle diameter of the inorganic particle powder (A) is preferably 0.15 to 1.0 μm, and more preferably 0.15 to 0.8 μm.

The shape of the inorganic particles (a) constituting the inorganic particle powder (A) is not particularly limited, and spherical, substantially spherical, or irregularly shaped particles can be used. However, from the viewpoint of excellent abrasion resistance and surface smoothness of the cured product of the dental curable composition, spherical or substantially spherical particles are preferable. The term "substantially spherical" refers to a particle having an average uniformity degree: Pr, of 0.6 or more. The average uniformity degree: Pr is defined by the formula: $Pr = (\Sigma B_i/L_i)/n$ based on sum of $B_i/L_i$ from the first to the n-th particle: $\Sigma B_i/L_i$. $B_i/L_i$ is the ratio of the minimum width: $B_i$ to the major axis: $L_i$, where the major axis: $L_i$ is the maximum length of each particle measured by image analysis performed on the 30 or more, preferably 100 or more of n inorganic primary particles selected from a captured image used when the average particle diameter is measured , and the minimum width: $B_i$ is a diameter in a direction orthogonal to the major axis. The average uniformity degree is preferably 0.7 or more, and particularly preferably 0.8 or more. The upper limit value of the average uniformity degree is 1.

The material of the inorganic particles (a) constituting the inorganic particle powder (A) is not particularly limited as long as the polarity of the zeta potential is negative, and examples thereof include metal oxides such as amorphous silica, quartz, titania, zirconia, chromium oxide, iron oxide, and tungsten oxide; and composite oxides such as silica-zirconia, silica-titania, silica-titania-barium oxide, silica-titania-zirconia, borosilicate glass, aluminosilicate glass, and fluoroaluminosilicate glass, which are used as fillers in conventional dental curable compositions.

For reasons of general usability and easy availability, the material of the inorganic particles (a) is preferably a silica-based inorganic compound. The silica-based inorganic compound means an inorganic compound containing silica. Among silica-based inorganic compounds, it is particularly preferable to use those composed of a silica-based composite oxide such as silica-zirconia, silica-titania, silica-titania-barium oxide, and silica-titania-zirconia, silica-zirconia being more preferable. Silica-based composite oxide particles often have strong acidic sites on their surface, and the negative degree of the zeta potential is often large.

The zeta potential of the inorganic particle powder (A) in water is preferably -20 mV or less, and more preferably -40 mV or less. The lower limit of the zeta potential is not particularly limited, but is generally -100 mV or more. The inorganic particle powder (A) may be a combination of a plurality of inorganic particle powders having different average primary particle diameters, and in this case, the zeta potential of each of the plurality of inorganic particle powders are preferably within the above-mentioned ranges.

In addition, the inorganic particle powder (A) may be surface-treated with a silane coupling agent or the like, as long as the polarity of the zeta potential is not changed.

The inorganic particle powder (B) in the present invention is composed of a plurality of the inorganic particles (b).

The average primary particle diameter of the inorganic particle powder (B) is 1 to 300 nm. By setting the average primary particle diameter of the inorganic particle powder (B), the average primary particle diameter of the inorganic particle powder (A), and the amount of each inorganic particle powder within specific ranges, it becomes easier to reduce the viscosity of the slurry used in producing the inorganic particle powder (C) of the present invention. Furthermore, as to be described later, when producing the inorganic particle powder (B) by mechanochemical treatment, particles with an average primary particle diameter of less than 1 nm are undesirable because the treatment takes a long time.

In addition, an average primary particle diameter of the inorganic particle powder (B) of 1 to 300 nm makes it easier to improve the polishability and transparency of the cured product of the dental curable composition.

From the viewpoint of reducing slurry viscosity and improving the polishability and transparency of the cured product of the dental curable composition, the average primary particle diameter of the inorganic particle powder (B) is preferably 15 to 280 nm, and more preferably 15 to 200 nm.

The average primary particle diameter of the inorganic particle powder (B) may be measured by the same method as the measurement method of the average primary particle diameter of the inorganic particle powder (A) described above.

The zeta potential of the inorganic particle powder (B) is positive (plus). The zeta potential of the inorganic particle powder (B) is preferably 10 mV or more, more preferably 20 mV or more, and is preferably 60 mV or less.

As for the material of the inorganic particles (b), rare earth metal compound particles are preferable from the viewpoint of color tone and safety. Among them, it is preferable to use crystalline rare earth metal fluorides such as ytterbium fluoride ($YbF_3$), lanthanum fluoride ($LaF_3$), cerium fluoride ($CeF_3$), and gadolinium fluoride ($GdF_3$), and it is most preferable to use ytterbium fluoride from the viewpoint of X-ray opacity. Whether it is crystalline can be determined by whether a peak based on a crystal plane is confirmed when X-ray diffraction measurement is performed.

In the present invention, when using the inorganic particle powder (B) composed of crystalline rare earth metal fluoride, it is preferable that the specific surface area is increased by pulverization or other methods. By increasing the specific surface area, the shapability of the dental curable composition, which is a paste, is improved. Here, shapability means the property of not deforming after the dental curable composition is discharged from the syringe and before it is cured (the property of being able to maintain its shape without deformation due to natural flow when left to stand).

The specific surface area of the inorganic particle powder (B) is preferably 10 to 100 $m^2/g$, more preferably 10 to 80 $m^2/g$, and even more preferably 10 to 70 $m^2/g$. The specific surface area can be measured by a nitrogen adsorption method.

Examples of a method for increasing the specific surface area of the inorganic particle powder (B) include a method of pulverizing a raw material particle powder (B') of the inorganic particle powder (B).

The pulverization method is not particularly limited, but mechanochemical treatment is preferred because a decrease in transparency when blended in a dental curable composition is suppressed and adjustment to transparency appropriate as a composite resin is facilitated. By mechanochemical treatment, it is possible to obtain a rare earth metal fluoride in which the specific surface area is increased and the amorphousness is enhanced at the same time.

Ytterbium fluoride ($YbF_3$) is well-known as an X-ray contrast filler, and it is known that when it is blended into a dental curable composition such as a composite resin, the transparency of the cured material is decreased. The decrease in transparency can be suppressed by enhancing the amorphousness by mechanochemical treatment.

The mechanochemical treatment refers to a treatment in which mechanical energy is applied to the raw material powder, and means a treatment in which at least one of mechanical grinding, pulverization, and dispersion is performed. From the viewpoint of reliably and efficiently increasing the specific surface area of the inorganic particle powder (B), it is preferable to adopt a wet method, and it is particularly preferable to perform a treatment using a wet beads mill (wet beads mill treatment).

Details of the wet beads mill treatment will be described later.

The mechanochemical treatment conditions vary depending on the conditions such as the operation method of the wet beads mill used, the bead diameter, the type of the inorganic particle powder, and the slurry concentration. The adjustment of these conditions can be performed by performing a preliminary experiment in an apparatus and conditions for actually performing the mechanochemical treatment, and confirming the specific surface area of the treated inorganic particle powder with respect to the mechanochemical treatment time.

In the case where the inorganic particle powder (B) composed of crystalline rare earth metal fluoride is obtained by the mechanochemical treatment, in addition to the aforementioned effect of increasing the specific surface area, the crystallinity of the crystalline rare earth metal fluoride decreases, suppressing the decrease in transparency when blended into the dental curable composition, and making it easier to adjust to transparency appropriate as a composite resin.

When using the inorganic particle powder (B) composed of crystalline rare earth metal fluoride, it is preferable that the full width at half maximum of the maximum intensity peak derived from the crystalline rare earth metal fluoride in an X-ray diffraction pattern obtained when X-ray diffraction measurement is performed on the inorganic particle powder (B) is 0.3° or more.

The crystallinity of the crystalline rare earth metal fluoride can be evaluated by the full width at half maximum of the maximum intensity peak derived from the crystalline rare earth metal fluoride in the X-ray diffraction pattern, and the smaller the full width at half maximum, the higher the crystallinity. Incidentally, the full width at half maximum of the maximum intensity peak of rare earth metal fluorides generally used as X-ray opaque materials and commercially available rare earth metal fluoride powders that can be obtained as raw material powders is usually less than 0.3° (specifically, about 0.12° to 0.27°), and when such a rare earth metal fluoride powder is blended, the transparency is decreased. In order to obtain the effect of suppressing the decrease in transparency, it is necessary to set the full width at half maximum of the maximum intensity peak derived from the crystalline rare earth metal fluoride in the X-ray diffraction pattern to 0.3° or more by performing, for example, mechanochemical treatment, and from the viewpoint of suppressing the decrease in transparency, the full width at half maximum of the maximum intensity peak is preferably 0.4° or more, and particularly preferably 0.5° or more.

The full width at half maximum of the maximum intensity peak increases as the mechanochemical treatment time increases, but the amount of increase varies depending on various conditions. Therefore, it is preferable to conduct preliminary experiments based on the apparatus and conditions for performing the mechanochemical treatment, and to confirm the full width at half maximum derived from the crystalline rare earth metal fluoride with respect to the mechanochemical treatment time. The upper limit of the full width at half maximum is not particularly limited, but in the case of mechanochemical treatment, the full width at half maximum usually does not exceed 2.0°.

The full width at half maximum of the maximum intensity peak in the X-ray diffraction pattern can be determined by performing X-ray diffraction measurement on the inorganic particle powder (B) composed of crystalline rare earth metal fluoride in the present invention. Specifically, the full width at half maximum can be determined by performing X-ray diffraction measurement in the range of $2\theta$; 20 to 120° with an X-ray diffraction apparatus, identifying the peak derived from the crystalline rare earth metal fluoride in the obtained X-ray diffraction pattern (chart) having $2\theta$ (°) on the horizontal axis and diffraction intensity on the vertical axis, and calculating the full width at half maximum of the peak having the maximum intensity (for example, for $YbF_3$, a peak corresponding to the crystal plane (1,1,1) appearing in the vicinity of $2\theta = 28.0°$), that is, the width of the peak at the intensity at which the intensity is 50% of the peak intensity (maximum intensity) (an absolute value of the difference of 2θ between two intersection points of the intensity and the peak line: unit "deg (°)"). In the measurement, it is preferable to use, as a measurement sample, a powder from which coarse grains are removed by, for example, using a sieve having an opening of 100 $\mu$m.

As described above, the inorganic particle powder (C) used in the present invention is composed of the aggregated particles (c) consisting of the inorganic particles (a) constituting the inorganic particle powder (A) and the inorganic particles (b) constituting the inorganic particle powder (B).

The average value of the total mass of the inorganic particles (b) relative to the total mass of 100 parts by mass of the inorganic particles (a) contained in each of the aggregated particles (c) that constitute the inorganic particle powder (C) is within the range of 20 to 300 parts by mass.

When the average value of the total mass of the inorganic particles (b) relative to the total mass of 100 parts by mass of the inorganic particles (a) exceeds 300 parts by mass, the dispersibility is poor when the inorganic particle powder (C) is blended into the dental curable composition, and aggregates are likely to form.

On the other hand, when the average value of the total mass of the inorganic particles (b) relative to the total mass of 100 parts by mass of the inorganic particles (a) is less than 20 parts by mass, it becomes difficult to produce the inorganic particle powder (C) by the spray drying method, and even if the inorganic particle powder (C) could be produced, the effect based on the blending of the inorganic particles (b) tends to decrease. For example, the shapability of the dental curable composition may decrease, and when the inorganic particles (b) have X-ray opacity, the X-ray contrast may easily decrease.

The average value of the total mass of the inorganic particles (b) relative to the total mass of 100 parts by mass of the inorganic particles (a) contained in each of the aggregated particles (c) is preferably 22 to 280 parts by mass, and more preferably 25 to 250 parts by mass.

The total mass of the inorganic particles (b) relative to the total mass of 100 parts by mass of the inorganic particles (a) can be adjusted by the amount of the inorganic particle powder (A) and the inorganic particle powder (B) used when producing the inorganic particle powder (C). That is, when producing the inorganic particle powder (C) by the spray drying method to be described later, it can be adjusted by the ratio of the amounts of the inorganic particle powder (A) and the inorganic particle powder (B) contained in the mixed slurry to be subjected to spray drying.

As described above, the inorganic particle powder (C) is an assembly of a plurality of the aggregated particles (c) with different particle diameters.

The average aggregated particle diameter of the inorganic particle powder (C) (i.e., the average value of the particle diameters of the plurality of the aggregated particles (c)) is 1 to 50 $\mu$m. The inorganic particle powder (C) having such an average aggregate particle diameter exhibit excellent operability. Furthermore, inorganic particle powder with an average aggregate particle diameter of less than 1 $\mu$m, or inorganic particle powder with an average aggregate particle diameter exceeding 50 $\mu$m, are difficult to prepare.

The average aggregate particle diameter of the inorganic particle powder (C) is preferably 3 to 30 $\mu$m, and more preferably 5 to 25 $\mu$m, from the viewpoint of improving operability.

The average aggregate particle diameter of the inorganic particle powder (C) can be adjusted to a desired range by adjusting producing conditions, such as the amount of a dispersion medium used in the spray drying method to be described later.

The average aggregate particle diameter of the inorganic particle powder (C) can be measured by a laser diffraction-scattering method.

It is preferable that the inorganic particle powder (C) does not contain an ionic surfactant. The inorganic particle powder (C), which does not contain an ionic surfactant, does not cause curing inhibition derived from the ionic surfactant when blended into the dental curable composition, discoloration due to the adsorption of pigments to the ionic surfactant, or a decrease in strength due to the elution of the ionic surfactant.

<Method for producing inorganic particle powder (C)>

The method for producing the inorganic particle powder (C) is not particularly limited, and it is preferable to produce the inorganic particle powder (C) by a spray drying method using a mixed slurry containing the inorganic particle powder (A) and the inorganic particle powder (B) described above. This will be described in detail below.

The inorganic particle powder (C) can be obtained through a process of spray drying a homogeneous mixed slurry obtained by mixing a slurry (Sa) in which 100 parts by mass of the inorganic particle powder (A) are dispersed in a dispersion medium, and a slurry (Sb) in which 20 to 300 parts by mass of the inorganic particle powder (B) are dispersed in a dispersion medium.

By a producing method including such a spray drying process, the inorganic particle powder (C) composed of the aggregated particles (c) in which the inorganic particles (b) are uniformly dispersed in the inorganic particles (a) can be obtained. Here, "uniformly dispersed" means that the inorganic particles (a) and the inorganic particles (b) are dispersed and mixed at the primary particle level in the aggregated particles (c), and, for example, when the inorganic particle powder (C) is observed with an electron microscope, the brightness of each constituent aggregated particle (c) is uniform, and particles with a specifically high brightness region, or particles having overall high brightness, such as aggregated particles consisting only of the inorganic particles (b), are not observed.

The mixed slurry is obtained by a process of preparing the slurry (Sa) and a process of preparing the slurry (Sb), and then mixing the slurry (Sa) and the slurry (Sb).

The process of preparing the slurry (Sa) is preferably carried out by subjecting a slurry obtained by mixing a dispersion medium and the inorganic particle powder (A) to a dispersion treatment. Water is preferably used as the dispersion medium; however, a mixture obtained by adding an organic solvent into water as needed may be used. Examples of the organic solvent include ethanol, isopropyl alcohol, chloroform, and dimethylformamide.

The amount of the dispersion medium used is usually 40 to 900 parts by mass with respect to 100 parts by mass of the inorganic particle powder (A).

The dispersion treatment can be performed using a mixing apparatus such as a beads mill.

As the inorganic particles (a) constituting the inorganic particle powder (A) contained in the slurry (Sa), a silica-based inorganic compound is preferred from the viewpoint of general usability and easy availability. Therefore, the slurry (Sa) is preferably a slurry in which the inorganic particles (a) composed of a silica-based inorganic compound are dispersed in water.

Among the above silica-based inorganic compounds, a silica-based composite oxide such as silica-zirconia, silica-titania, silica-titania-barium oxide, and silica-titania-zirconia is particularly preferred.

The process of preparing the slurry (Sb) is preferably carried out by subjecting a slurry containing a dispersion medium and the inorganic particle powder (B) to a dispersion treatment. Water is preferably used as the dispersion medium; however, a mixture obtained by adding an organic solvent into water as needed may be used. Examples of the organic solvent include ethanol, isopropyl alcohol, chloroform, and dimethylformamide.

The amount of the dispersion medium used is usually 40 to 900 parts by mass with respect to 100 parts by mass of the inorganic particle powder (B).

The dispersion treatment can be performed using a mixing apparatus such as a beads mill.

As the inorganic particles (b) constituting the inorganic particle powder (B) contained in the slurry (Sb), crystalline rare earth metal fluorides are preferred from the viewpoint of color tone and stability. Therefore, the slurry (Sb) is preferably a slurry in which the inorganic particles (b) composed of crystalline rare earth metal fluorides are dispersed in water.

Among the above crystalline rare earth metal fluorides, it is preferable to use ytterbium fluoride ($YbF_3$), lanthanum fluoride ($LaF_3$), cerium fluoride ($CeF_3$), and gadolinium fluoride ($GdF_3$), and it is most preferable to use ytterbium fluoride from the viewpoint of X-ray opacity.

When using the inorganic particle powder (B) composed of crystalline rare earth metal fluorides, the process of preparing the slurry (Sb) preferably involves a wet beads mill treatment of the raw material particle powder (B') using water as a medium (dispersion medium). The raw material particle powder (B') is composed of crystalline rare earth metal fluoride particles, and the full width at half maximum of the maximum intensity peak derived from the crystalline rare earth metal fluoride in the X-ray diffraction pattern obtained when X-ray diffraction measurement is performed is less than 0.3°.

By the wet beads mill treatment of the raw material particle powder (B'), it is possible to obtain the slurry (Sb) in which the inorganic particle powder (B) is dispersed, where the full width at half maximum of the maximum intensity peak derived from the crystalline rare earth metal fluoride in the X-ray diffraction pattern obtained when X-ray diffraction measurement is performed is 0.3° or more.

In such an inorganic particle powder of the present invention, produced using the slurry (Sb) in which the inorganic particle powder (B) is dispersed, a decrease in transparency when blended in a dental curable composition is suppressed and adjustment to transparency appropriate as a composite resin is facilitated.

The wet beads mill treatment is one of the mechanochemical treatment, and is a processing method in which a slurry obtained by mixing a powder to be processed and a medium (dispersion medium) is brought into contact with a medium (beads) that has been moved by stirring or vibration, thereby imparting a pulverization and disintegration action to the powder. Examples of a material used as a medium include glass, alumina, zircon, zirconia, steel, and resin, and it is preferable to use beads made of alumina or zirconia because the beads have excellent abrasion resistance and relatively little contamination. The size of the beads to be used may be selected according to the average particle diameter of the target inorganic particle powder (B), and is not particularly limited, but in order to obtain the inorganic particle powder (B) preferable for blending into the dental curable composition, beads having a diameter of 0.01 to 0.5 mm are preferably used.

The wet bead mill is classified into a batch type in which a slurry and beads are directly charged into an apparatus and treated, a circulation type in which a slurry is circulated between a tank and an apparatus, and a pass type in which a slurry is passed through an apparatus a predetermined number of times, depending on the operation method, and these operation methods may be selected depending on the amount of the inorganic particle powder (B) to be used for the treatment. It is preferable to use a circulation type bead mill or a pass type bead mill because they have good productivity and can treat a relatively large amount of inorganic particle powder.

Depending on the operation method such as the circulation type and the pass type, it is necessary to separate the slurry and the beads when the mechanochemical treatment is carried out. Examples of the bead separation method include a slit type, a screen type, and a centrifugal separation type. These bead separation methods may be selected depending on the particle diameter of the beads to be used, and any method can be used without particular limitation. The concentration of the slurry subjected to the mechanochemical treatment is usually 40 to 900 parts by mass of the dispersion medium with respect to 100 parts by mass of the raw material particle powder (B').

The amount of the inorganic particle powder (B) contained in the slurry (Sb) is preferably 20 to 300 parts by mass, more preferably 22 to 280 parts by mass, and even more preferably 25 to 250 parts by mass, with respect to 100 parts by mass of the inorganic particle powder (A) contained in the slurry (Sa).

By mixing the slurry (Sa) and the slurry (Sb) prepared as described above, a homogeneous mixed slurry is obtained. Mixing can be performed using a stirrer or the like.

The mixed slurry preferably does not contain an ionic surfactant. In the present invention, even without using an ionic surfactant, the viscosity of the mixed slurry can be lowered, resulting in a slurry suitable for spray drying, which will be described later.

Furthermore, because the mixed slurry does not contain an ionic surfactant, an inorganic particle powder (C) that does not contain an ionic surfactant can be obtained. Such an inorganic particle powder (C), which does not contain an ionic surfactant, does not cause curing inhibition derived from the ionic surfactant when blended into the dental curable composition, discoloration derived from the adsorption of pigments to the ionic surfactant, or a decrease in strength derived from the elution of the ionic surfactant.

Here, examples of the ionic surfactant include an anionic surfactant, a cationic surfactant, and an amphoteric surfactant.

A surfactant is a compound that has both a hydrophilic group and a hydrophobic group in a single molecule. An anionic surfactant is a surfactant that can become ions in an aqueous solution, with the hydrophilic group becoming an anion. A cationic surfactant is a surfactant that can become ions in an aqueous solution, with the hydrophilic group becoming a cation. An amphoteric surfactant is a compound that, when dissolved in water, exhibits the properties of an anionic surfactant in an alkaline region and the properties of a cationic surfactant in an acidic region.

Furthermore, the mixed slurry may contain a surface treatment agent as needed. By using a surface treatment agent, the inorganic particles constituting the inorganic particle powder (A) or the inorganic particle powder (B) can be surface-treated.

Examples of such a surface treatment agent include a silane coupling agent, such as vinyltriethoxysilane, vinyltrimethoxysilane, vinyltris(β-methoxyethoxy)silane, γ-methacryloyloxypropyltrimethoxysilane, κ-methacryloyloxydodecyltrimethoxysilane, β-(3,4-epoxycyclohexyl)-ethyltrimethoxysilane, γ-glycidoxypropyl-trimethoxysilane, N-β-(aminoethyl)-γ-aminopropyl-trimethoxysilane, γ-ureidopropyl-triethoxysilane, γ-chloropropyltrimethoxysilane, methyltrimethoxysilane, ethyltrimethoxysilane, and methyltriethoxysilane, and a titanate coupling agent.

The amount of the surface treatment agent blended is, for example, 0.1 to 10 parts by mass, and preferably 0.5 to 5 parts by mass, with respect to a total of 100 parts by mass of the inorganic particle powder (A) and the inorganic particle powder (B).

The inorganic particle powder (C) can be obtained by spray drying the mixed slurry.

As the spray drying method, a method of atomizing the mixed slurry into fine droplets using a high-speed airflow and drying the slurry (also called a nozzle-type spray drying method), or a method of dropping the mixed slurry onto a disc-shaped rotating body rotating at a rotational speed of 1000 to 50000 rpm, and then centrifugally throwing the slurry off in a mist form and then drying the slurry (also called a disc-type spray drying method) may be employed. Although the resulting inorganic particle powder (C) is composed of the aggregated particles (c), from the viewpoint of obtaining the aggregated particles (c) with uniform particle size, the disc-type spray drying method, which immediately dries the atomized mixed slurry with high-temperature air or inert gas, is preferred. In this case, the temperature of the gas used for drying is preferably 60 to 300°C, and particularly preferably 80 to 250°C.

Furthermore, the disc-type spray drying method allows for appropriate spray drying even when the concentration of the mixed slurry is relatively high.

The concentration of the mixed slurry used in spray drying is preferably less than 40% by mass, more preferably 38% by mass or less, and even more preferably 35% by mass or less, from the viewpoint of appropriately performing spray drying and obtaining desired aggregated particles, and is preferably 10% by mass or more from the viewpoint of improving the productivity of the inorganic particle powder (C).

The concentration of the mixed slurry refers to the concentration (% by mass) of inorganic particle powder in the mixed slurry.

The inorganic particle powder (C) obtained by spray drying is preferably subjected to vacuum drying after spray drying, from the viewpoint of removing a residual dispersion medium. Vacuum drying is generally performed under reduced pressure of 0.01 to 100 hectopascals at 20 to 150°C for 1 to 48 hours.

The inorganic particle powder obtained by spray drying may be pulverized as needed to be adjusted to an appropriate average particle diameter. As pulverizing methods, a vibration ball mill, a bead mill, a jet mill, etc., can be used.

<Inorganic particle powder (A), Organic-inorganic composite particle powder (D)>

The dental curable composition of the present invention further contains at least one of the inorganic particle powder (A) and an organic-inorganic composite particle powder (D) as a particle powder different from the above-mentioned inorganic particle powder (C), from the viewpoint of improving the mechanical strength of the cured product.

The details of the structure of the inorganic particle powder (A) are as described above, so the description here is omitted.

The organic-inorganic composite particle powder (D) is composed of a plurality of organic-inorganic composite particles (d). The organic-inorganic composite particles (d) consist of a composite material, which is a composite material of the inorganic particle powder (A) and a resin, and in which the inorganic particle powder (A) has a content of 60 to 90% by mass in the composite material. The content of the inorganic particle powder (A) in the composite material is preferably 65 to 90% by mass, and more preferably 70 to 90% by mass.

When using the organic-inorganic composite particle powder (D), the inorganic particle powder (A) can be blended into the composition as the organic-inorganic composite particle powder (D). In the organic-inorganic composite particle powder (D), the inorganic particle powder (A) is coated with resin, which tends to be less likely to interact with the inorganic particle powder (B).

Although the resin in the above composite material is not particularly limited, it is preferably a cured product obtained by polymerizing a polymerizable monomer. As the polymerizable monomer, those described as the polymerizable monomer (M) can be used without any particular limitations.

The average particle diameter of the organic-inorganic composite particle powder (D) is 1 to 100 $\mu$m. When the average particle diameter is less than 1 $\mu$m, the discharging properties of the dental curable composition when discharged from a syringe deteriorate. When the average particle diameter exceeds 100 $\mu$m, the mechanical strength, such as the flexural strength, of the cured product of the dental curable composition tends to decrease.

The average particle diameter of the organic-inorganic composite particle powder (D) corresponds to the average value of the particle diameters of the plurality of the organic-inorganic composite particles (d), and is defined as a median diameter in a volume-based particle size distribution measured by a laser diffraction-scattering method.

The organic-inorganic composite particle powder (D) may be obtained by polymerizing a mixture of the inorganic particle powder (A), a polymerizable monomer, and a polymerization initiator, and then pulverizing the resulting product. Alternatively, the organic-inorganic composite particle powder (D) may be a microporous organic-inorganic composite particle powder obtained by immersing an aggregated particle powder composed of inorganic aggregated particles, which are aggregates of the inorganic particles (a) constituting the inorganic particle powder (A), in a polymerizable monomer solution containing a polymerizable monomer, a polymerization initiator, and an organic solvent, then removing the organic solvent, and subsequently polymerizing and curing the polymerizable monomer. The above aggregated particle powder can be obtained, for example, by spray-drying an aqueous dispersion containing the inorganic particle powder (A).

As the polymerizable monomer, any of those described as the polymerizable monomer (M) can be used without any particular limitations, and as the polymerization initiator, any of those described later suitable to be added to the dental curable composition can be used without any particular limitations.

<Content of inorganic particle powder (A) and inorganic particle powder (B) regardless of the form of inclusion>

As described above, the inorganic particle powder (C) is composed of a plurality of aggregated particles (c), the organic-inorganic composite particle powder (D) is composed of a plurality of organic-inorganic composite particles (d), and the inorganic particle powder (A) is composed of a plurality of inorganic particles (a) as described above. On the other hand, since the inorganic particle powder (A) is used as a raw material in the production of the inorganic particle powder (C) and the organic-inorganic composite particle powder (D) as described above, the inorganic particle powder (C) and the organic-inorganic composite particle powder (D) each contain the inorganic particle powder (A). Therefore, when the inorganic particle powder (A) is blended as a particle powder other than the inorganic particle powder (C) and the organic-inorganic composite particle powder (D), the dental curable composition will contain the inorganic particle powder (A) in different forms of inclusion. For this reason, the term "the total content of the inorganic particle powder (A) regardless of the form of inclusion", which represents the content of all inorganic particle powder (A) in the dental curable composition (that is, the total amount of the inorganic particle powder (A), including the amount of the inorganic particle powder (A) contained in the inorganic particle powder (C) and the amount of the inorganic particle powder (A) contained in the organic-inorganic composite particle powder (D) that is blended as needed), is used.

In the dental curable composition, the total content of the inorganic particle powder (A), regardless of its form of inclusion, is 170 to 270 parts by mass, and more preferably 180 to 250 parts by mass, with respect to 100 parts by mass of the polymerizable monomer (M). When the total content of the inorganic particle powder (A), regardless of its form of inclusion, is less than 170 parts by mass, the change in fluidity of the dental curable composition over time tends to increase, and the mechanical strength of the cured product tends to decrease. When the total content of the inorganic particle powder (A), regardless of its form of inclusion, exceeds 270 parts by mass, the discharging properties deteriorate.

Furthermore, since the inorganic particle powder (B) is used as a raw material in the production of inorganic particle powder (C), the inorganic particle powder (C) will contain the inorganic particle powder (B). In addition, the dental curable composition of the present invention may also contain the inorganic particle powder (B) as a particle powder other than the inorganic particle powder (C). In such cases, the dental curable composition will contain the inorganic particle powder (B) in different forms of inclusion. For this reason, the term "total content of the inorganic particle powder (B) regardless of the form of inclusion", which represents the content of all inorganic particle powders (B) in the dental curable composition (that is, the total amount of the inorganic particle powder (B) contained in the inorganic particle powder (C) and the inorganic particle powder (B) in other forms of inclusion that is blended as needed), is used.

In the dental curable composition, the total content of the inorganic particle powder (B), regardless of its form of inclusion, is 5 to 50 parts by mass, and preferably 8 to 45 parts by mass, with respect to 100 parts by mass of the polymerizable monomer (M), from the viewpoint of improving the mechanical strength of the cured product.

When the total content of the inorganic particle powder (B) regardless of its form of inclusion is less than 5 parts by mass, the change in fluidity over time becomes large, and the shapability deteriorates. Furthermore, when the total content of the inorganic particle powder (B) regardless of its form of inclusion exceeds 50 parts by mass, the discharging properties deteriorate.

< Polymerization initiator>

A polymerization initiator may be added to the dental curable composition of the present invention. The polymerization initiator is not particularly limited as long as it has a function of polymerizing the polymerizable monomer, and it is preferable to use a photopolymerization initiator or a chemical polymerization initiator used in dental direct filling and restoration applications in which curing is often performed in the oral cavity, and it is more preferable to use a photopolymerization initiator from the viewpoint of simplicity without the need for a mixing operation.

As the polymerization initiator used for photopolymerization, benzoin alkyl ethers such as benzoin methyl ether, benzoin ethyl ether, and benzoin isopropyl ether; benzil ketals such as benzil dimethyl ketal and benzil diethyl ketal; benzophenones such as benzophenone, 4,4'-dimethylbenzophenone, and 4-methacryloxybenzophenone; $\alpha$-diketones such as diacetyl, 2,3-pentanedione benzyl, camphorquinone, 9,10-phenanthraquinone, and 9,10-anthraquinone; thioxanthone compounds such as 2,4-diethoxythioxanthone, 2-chlorothioxanthone, and methylthioxanthone; and bisacylphosphine oxides such as bis-(2,6-dichlorobenzoyl)phenylphosphine oxide, bis-(2,6-dichlorobenzoyl)-2,5-dimethylphenylphosphine oxide, bis-(2,6-dichlorobenzoyl)-4-propylphenylphosphine oxide, bis-(2,6-dichlorobenzoyl)-1-naphthylphosphine oxide, and bis (2,4,6-trimethylbenzoyl)phenylphosphine oxide can be used.

In addition, a reducing agent is often added to the photopolymerization initiator, and examples thereof include tertiary amines such as 2-(dimethylamino)ethyl methacrylate, ethyl 4-dimethylaminobenzoate, and N-methyldiethanolamine; aldehydes such as lauryl aldehyde, dimethylaminobenzaldehyde, and terephthalaldehyde; and sulfur-containing compounds such as 2-mercaptobenzoxazole, 1-decanethiol, thiosalicylic acid, and thiobenzoic acid.

Further, in many cases, a photoacid generator is used in addition to the photopolymerization initiator and the reducing agent. Examples of such a photoacid generator include a diaryliodonium salt-based compound, a sulfonium salt-based compound, a sulfonic acid ester compound, a halomethyl-substituted-S-triazine derivative, and a pyridinium salt-based compound.

These polymerization initiators may be used alone or as a mixture of two or more thereof. The blending amount of the polymerization initiator may be selected as an effective amount depending on the purpose, and is usually the proportion of 0.01 to 10 parts by mass, and more preferably the proportion of 0.1 to 5 parts by mass with respect to 100 parts by mass of the polymerizable monomer.

<Other Additives>

In the dental curable composition of the present invention, additives such as a polymerization inhibitor, a pigment, an ultraviolet absorber, and a fluorescent agent can be blended within a range that does not impair the effects.

The dental curable composition of the present invention can be prepared by mixing the polymerizable monomer (M), the inorganic particle powder (C), and at least one of the inorganic particle powder (A) and the organic-inorganic composite particle powder (D), along with optional components blended as needed, in predetermined amounts to obtain a paste, and then defoaming the paste under reduced pressure to remove air bubbles.

The dental curable composition of the present invention has good discharging properties from a syringe, appropriate fluidity, little change in fluidity over time, and high mechanical strength after curing. Therefore, it is suitable for use as a flowable composite resin.

[Inorganic Particle powder (C)]

In the present invention, the inorganic particle powder (C) can be provided as an inorganic particle powder that exhibits good dispersibility when blended into the dental curable composition.

The inorganic particle powder (C) is composed of aggregated particles (c) consisting of inorganic particles (a) that constitute an inorganic particle powder (A) having an average primary particle diameter of 50 nm to 1 $\mu$m as measured by electron microscopy, the inorganic particle powder (A) composed of a plurality of inorganic particles (a) composed of the same type of material, exhibiting a negative zeta potential polarity when measured in water, and inorganic particles (b) that constitute an inorganic particle powder (B) having an average primary particle diameter of 1 to 300 nm as measured by electron microscopy, the inorganic particle powder (B) composed of a plurality of inorganic particles (b) composed of the same type of material, exhibiting a positive zeta potential polarity when measured in water, in which,

an average value of a total mass of the inorganic particles (b) relative to a total mass of 100 parts by mass of the inorganic particles (a) contained in each of the aggregated particles (c) that constitute the inorganic particle powder (C) being within a range of 20 to 300 parts by mass,
the inorganic particle powder having an average aggregate particle diameter of 1 to 50 $\mu$m, defined as a median diameter in a volume-based particle size distribution measured by a laser diffraction-scattering method, and being free of an ionic surfactant.

The details of the inorganic particle powder (C) and the method for producing the inorganic particle powder (C) are as described above for the inorganic particle powder (C) contained in the dental curable composition, so the description here is omitted.

Examples

Hereinafter, the present invention will be specifically described with reference to Examples and Comparative Examples. However, the present invention is not limited to these Examples.

First, in Examples and Comparative Examples, substances used as raw materials of the compositions to be prepared and abbreviations thereof, and evaluation methods of the above-mentioned raw materials and prepared compositions will be described.

1. Raw materials and abbreviations thereof

(1) Polymerizable monomers

- UDMA: 1,6-bis(methacrylethyloxycarbonylamino)-2,2-4-trimethylhexane
- 3G: triethylene glycol dimethacrylate
- GMA: 2,2-bis[(3-methacryloyloxy-2-hydroxypropyloxy)phenyl]propane
- D-2.6E: 2,2-bis(4-methacryloyloxypolyethoxyphenyl)propane

(2) Inorganic particle powder

The average primary particle diameter and the average uniformity degree are values determined based on an evaluation method to be described later with respect to each inorganic particle powder.

(2)-1 Inorganic particle powder composed of silica-based inorganic compounds

· FA-1: inorganic particle powder composed of spherical silica-zirconia particles produced by a sol-gel method (average primary particle diameter: 150 nm, average uniformity degree: 0.95)
· FA-2: inorganic particle powder composed of spherical silica-zirconia particles produced by a sol-gel method (average primary particle diameter: 260 nm, average uniformity degree: 0.95)
· FA-3: inorganic particle powder composed of spherical silica-zirconia particles produced by a sol-gel method (average primary particle diameter: 400 nm, average uniformity degree: 0.9)
· FA-4: inorganic particle powder composed of irregularly shaped silica-zirconia particles produced by a sol-gel method (average primary particle diameter: 1000 nm)

· FA-5: inorganic particle powder composed of silica (average primary particle diameter: 300 nm, manufactured by Nippon Shokubai Co., Ltd.)

· FA-6: inorganic particle powder composed of spherical silica-zirconia particles produced by a sol-gel method (average primary particle diameter: 40 nm, average uniformity degree: 0.90)

· FA-7: inorganic particle powder composed of irregularly shaped silica-zirconia produced by a sol-gel method (average primary particle diameter: 4000 nm).

(2)-2 Inorganic particle powder composed of crystalline rare earth fluoride particles

· $YbF_3$-40: inorganic particle powder composed of ytterbium trifluoride (average primary particle diameter: 46 nm, manufactured by Treibacer Industrie AG).

· $YbF_3$-200: inorganic particle powder composed of ytterbium trifluoride (average primary particle diameter 200 nm, manufactured by Treibacer Industrie AG).

· $YbF_3$-300: inorganic particle powder composed of ytterbium trifluoride (average primary particle diameter 270 nm, manufactured by Treibacer Industrie AG).

(3) Polymerization initiator

· CQ: camphorquinone

· DMBE: ethyl N,N-dimethyl-p-benzoate.

2. Producing method, evaluation method, and evaluation result of inorganic particle powder

(1) Inorganic particle powder (A) Composed of Silica-Based Inorganic Compound

The average primary particle diameter and the zeta potential of the inorganic particle powders FA-1 to FA-7 were determined as follows. The results are shown in Table 1. Furthermore, a slurry prepared by mixing 400 parts by mass of each inorganic particle powder with 600 parts by mass of ion-exchanged water was subjected to dispersion treatment using a wet beads mill SC50 (manufactured by Mitsui Mining Co., Ltd.) at a rotation speed of 3000 rpm for 10 minutes using 100 g of φ0.3 mm zirconia beads as a medium, to prepare slurries (SA-1 to SA-7) in which the inorganic particle powders composed of silica-based inorganic compounds were dispersed.

Slurry SA-1 is a slurry in which the inorganic particle powder FA-1 is dispersed, slurry SA-2 is a slurry in which the inorganic particle powder FA-2 is dispersed, slurry SA-3 is a slurry in which the inorganic particle powder FA-3 is dispersed, slurry SA-4 is a slurry in which the inorganic particle powder FA-4 is dispersed, slurry SA-5 is a slurry in which the inorganic particle powder FA-5 is dispersed, slurry SA-6 is a slurry in which the inorganic particle powder FA-6 is dispersed, and slurry SA-7 is a slurry in which the inorganic particle powder FA-7 is dispersed.

<Method for measuring average primary particle diameter>

A photograph of the powder was taken at a magnification of 5,000 to 100,000 times using a scanning electron microscope ("XL-30S" manufactured by Philips), and the image was processed using image analysis software ("IP-1000PC" manufactured by Asahi Kasei Engineering Corporation), and the average primary particle diameter was determined based on the measured value of the number (100 or more) of particles observed in the unit field of view of the photograph.

<Method for measuring zeta potential of inorganic particle powder>

The inorganic particle powders were suspended in ion-exchanged water having a pH of 7, and dispersed in water by being irradiated with ultrasonic waves for 30 minutes so that the concentration of the suspended water was 1.0% by mass. The zeta potential of the suspended water was measured using a zeta potential measuring apparatus ("ELSZ-2000" manufactured by Otsuka Electronics Co., Ltd.). The measurement was performed three times for each sample, and the average thereof was defined as the zeta potential.

[Table 1]

|  | Material | Average primary particle diameter (nm) | Shape | Zeta potential (mV) |
|---|---|---|---|---|
| FA-1 | Silica-zirconia | 150 | Spherical | -44 |
| FA-2 | Silica-zirconia | 260 | Spherical | -41 |
| FA-3 | Silica-zirconia | 400 | Spherical | -38 |
| FA-4 | Silica-zirconia | 1000 | Irregularly shaped | -32 |
| FA-5 | Silica | 300 | Spherical | -25 |
| FA-6 | Silica-zirconia | 40 | Spherical | -46 |
| FA-7 | Silica-zirconia | 4000 | Irregularly shaped | -18 |

(2) Inorganic particle powder (B) composed of crystalline rare earth metal fluoride

Each of the inorganic particle powders composed of the crystalline rare earth metal fluoride ($YbF_3$-40, $YbF_3$-200, and $YbF_3$-300) was subjected to mechanochemical treatment to obtain slurries (SB-1 to SB-5) in which the inorganic particle powder (B) was dispersed.

The mechanochemical treatment was performed by subjecting a slurry obtained by mixing 400 parts by mass of each of the inorganic particle powders composed of crystalline rare earth fluoride with 600 parts by mass of ion-exchanged water to dispersion treatment using a wet beads mill SC50 (manufactured by Nippon Coke & Engineering Co., Ltd.) at a rotation speed of 3000 rpm for a treatment time shown in Table 2 using 100 g of φ0.3 mm zirconia beads as a medium.

The obtained slurries were dried under reduced pressure using an evaporator to prepare the inorganic particle powder (B): FB-1 to FB-5, with the material of the crystalline rare earth metal fluoride particles and the treatment time of the dispersion treatment as shown in Table 2. Regarding the inorganic particle powders thus obtained, the average primary particle diameter and zeta potential were measured in the same manner as in the above (1), and 2θ of the peak of the crystal plane (1,1,1) in the X-ray diffraction pattern, and the full width at half maximum (°) were measured as follows. The results are shown in Table 2.

< Method for measuring 2θ and full width at half maximum (°) of crystal plane (1,1,1)>

The particle powder was filled into a sample stage, and an X-ray diffraction pattern (chart) was obtained by performing measurement using an X-ray diffraction apparatus ("Smartlab" manufactured by Rigaku Corporation), in which the horizontal axis represents 2θ (°) and the vertical axis represents diffraction intensity. Here, CuKα radiation was used as the X-ray for the X-ray diffraction measurement.

When the material of the crystalline rare earth metal fluoride particles is $YbF_3$, the peak with the highest intensity is a peak attributed to the (1,1,1) plane (the peak observed around 2θ = 28°), so the full width at half maximum (°) was determined for this peak.

[Table 2]

|  | Slurry Abbreviation | Material | SC mill treatment time (min.) | Average primary particle diameter (nm) | Zeta potential (mV) | Maximum peak (1,1,1) | |
|---|---|---|---|---|---|---|---|
|  |  |  |  |  |  | 2θ (°) | Full width at half maximum (°) |
| FB-1 | SB-1 | YbF3-40 | 600 | 15 | +50 | 28 | 0.77 |
| FB-2 | SB-2 | YbF3-40 | 300 | 28 | +49 | 28 | 0.59 |
| FB-3 | SB-3 | YbF3-200 | 300 | 84 | +49 | 27.9 | 0.43 |
| FB-4 | SB-4 | YbF3-300 | 300 | 136 | +49 | 27.8 | 0.41 |
| FB-5 | SB-5 | YbF3-300 | 0 | 270 | +48 | 27.9 | 0.22 |

(3) Preparation of inorganic particle powder (C) composed of aggregated particles

(FC-1 to FC-16)

100 g of slurry SA-2 and 25 g of slurry SB-2 were mixed to obtain a mixed slurry. Then, 1.6 g (0.006 mol) of $\gamma$-methacryloyloxypropyltrimethoxysilane and 20 g of water were added, followed by the addition of acetic acid to adjust the pH to 4, and the mixture was stirred for 1 hour and 30 minutes to obtain a homogeneous solution. This solution and ion-exchanged water (50 g) for concentration adjustment were added to the mixed slurry and mixed uniformly. Thereafter, while gently mixing the dispersion, the inorganic powder dried using a spray drying method was collected from a cyclone recovery section and a main body lower recovery section. The spray dryer used was a spray dryer (spray dryer "FOC-20", manufactured by Okawara Kakoki Co., Ltd.). The disk rotation speed was 26,000 rpm, and the temperature of the drying atmosphere air was 200°C. Subsequently, the inorganic powder collected from the cyclone recovery section was vacuum-dried at 80°C for 17 hours to obtain the inorganic particle powder (C): FC-1. Similarly, the inorganic powder collected from the main body lower recovery section was vacuum-dried to obtain the inorganic particle powder (C): FC-2.

As shown in Table 3, the inorganic particle powder (C): FC-3 to FC-13 were prepared in the same manner as FC-1, except that the types and ratios of the silica-based inorganic compound slurry and the crystalline rare earth metal fluoride slurry used in the preparation of the inorganic particle powder (C), and the amount of ion-exchanged water added for concentration adjustment were changed.

When using slurries (SA-6, SA-7) in which the inorganic particle powders (FA-6, FA-7) composed of silica-based composite oxides that do not meet the requirements of the present invention are dispersed, or when the amount of crystalline rare earth metal fluoride is small and the viscosity of the slurry is high, the slurry clogged the nozzle during spray drying, making spraying impossible (FC-14 to FC-16).

The average aggregate particle diameter (median diameter in a volume-based particle size distribution) of the obtained inorganic particle powder (C) was measured as follows.

The formation of aggregated particles in the inorganic particle powder (C) was confirmed by scanning electron microscopy.

(FC-17)

100 g of slurry SA-2 and 100 g of slurry SB-2 were mixed to obtain a mixed slurry. Then, 1.6 g (0.006 mol) of $\gamma$-methacryloyloxypropyltrimethoxysilane and 20 g of water were added, followed by the addition of acetic acid to adjust the pH to 4, and the mixture was stirred for 1 hour and 30 minutes to obtain a homogeneous solution. This solution and 100 g of ion-exchanged water for concentration adjustment were added to the mixed slurry and mixed uniformly. Thereafter, while gently mixing the dispersion, the inorganic particle powder (C) dried using a spray drying method was collected from a cyclone recovery section and a main body lower recovery section.

The spray dryer used was a spray dryer (spray dryer "RL-8", manufactured by Okawara Kakoki Co., Ltd.). The spray pressure was 0.20 MPa, and the temperature of the drying atmosphere air was 200°C. Subsequently, the inorganic powder collected from the cyclone recovery section was vacuum-dried at 80°C for 17 hours to obtain the inorganic particle powder (C): FC-17, composed of 65 g of aggregated particles.

The average aggregate particle diameter (median diameter in a volume-based particle size distribution) of the obtained inorganic particle powder (C) was measured as follows.

The formation of aggregated particles in the inorganic particle powder (C) was confirmed by scanning electron microscopy.

<Method for measuring average aggregate particle diameter>

0.1g of the inorganic particle powder composed of aggregated particles was dispersed in 10 mL of ethanol and thoroughly shaken by hand. The median diameter of the volume statistics was determined by applying an optical model "Fraunhofer" using a laser diffraction-scattering particle size distribution analyzer ("LS230", manufactured by Beckman Coulter).

The average value of the total mass of crystalline rare earth metal fluoride (the inorganic particles (b)) with respect to a total mass of 100 parts by mass of the silica-based inorganic compound (the inorganic particles (a)) contained in each of the aggregated particles constituting the obtained inorganic particle powder (FC-1) was 25 parts by mass, corresponding to the value shown in Table 3. Similarly, the average values of the total mass of crystalline rare earth metal fluoride (the inorganic particles (b)) with respect to 100 parts by mass of a total mass of 100 parts by mass of the silica-based inorganic compound (the inorganic particles (a)) for the other inorganic particle powders (FC-2 to FC17) correspond to the values listed in Table 3.

The concentrations (% by mass) listed in Table 3 refer to the concentration (% by mass) of the inorganic particle powder in the mixed slurry used in spray drying.

[Table 3]

| | Slurry of silica-based inorganic compound (parts by mass) | | | | | | | Slurry of crystalline rare earth metal fluoride (parts by mass) | | | | | Amount of ionexchanged water added (g) | Concentration (% by mass) | Average aggregate particle diameter (μm) |
|---|---|---|---|---|---|---|---|---|---|---|---|---|---|---|---|
| | SA-1 | SA-2 | SA-3 | SA-4 | SA-5 | SA-6 | SA-7 | SB-1 | SB-2 | SB-3 | SB-4 | SB-5 | | | |
| FC-1 | | 100 | | | | | | | 25 | | | | 50 | 25 | 16 |
| FC-2 | | 100 | | | | | | | 25 | | | | 50 | 25 | 42 |
| FC-3 | | 100 | | | | | | | | 70 | | | 20 | 32 | 18 |
| FC-4 | | 100 | | | | | | | | | 100 | | 0 | 36 | 19 |
| FC-5 | | 100 | | | | | | | 250 | | | | 0 | 38 | 23 |
| FC-6 | | 100 | | | | | | 100 | | | | | 400 | 13 | 7 |
| FC-7 | | 100 | | | | | | | | | | 100 | 0 | 36 | 19 |
| FC-8 | | 100 | | | | | | | | | | 100 | 40 | 31 | 18 |
| FC-9 | 100 | | | | | | | | 150 | | | | 30 | 33 | 14 |
| FC-10 | | | 100 | | | | | | 100 | | | | 30 | 32 | 17 |
| FC-11 | | | | 100 | | | | | 100 | | | | 30 | 32 | 17 |
| FC-12 | | | | | 100 | | | | 100 | | | | 100 | 25 | 18 |
| FC-13 | | 100 | | | | | | | 400 | | | | 0 | 38 | 19 |
| FC-14 | | 100 | | | | | | | 10 | | | | 0 | 33 | Spray not possible |
| FC-15 | | | | | | 100 | | | 100 | | | | 0 | 36 | Spray not possible |
| FC-16 | | | | | | | 100 | | 30 | | | | 0 | 34 | Spray not possible |
| FC-17 | | 100 | | | | | | | 100 | | | | 100 | 25 | 3 |

18

EP 4 778 504 A1

(4) Organic-inorganic composite particle powder (D) composed of organic-inorganic composite particles consisting of composite material of resin and inorganic particle powder (A) composed of silica-based composite oxide

100 g of the inorganic particle powder (FA-2) was added to 200 g of ion-exchanged water, and an aqueous dispersion of thereof (dispersion of inorganic particle powder) was obtained using a circulating pulverizer SC mill (manufactured by Nippon Coke Industries Co., Ltd.). Next, 4 g (0.016 mol) of $\gamma$-methacryloyloxypropyltrimethoxysilane and 0.003 g of acetic acid were added to 80 g of water, and the mixture was stirred for 1 hour and 30 minutes to obtain a homogeneous solution with a pH of 4. This solution was added to the above-mentioned dispersion of inorganic particle powder and mixed until homogeneous. Thereafter, while gently mixing the dispersion, it was supplied onto a high-speed rotating disk and granulated by a spray drying method. Spray drying was performed using a spray dryer TSR-2W (manufactured by Sakamoto Giken Co., Ltd.) equipped with a rotating disk, which atomizes the material by centrifugal force. The disk rotation speed was 10,000 rpm, and the temperature of the drying atmosphere air was 200°C. Subsequently, the powder obtained by granulation through spray drying was vacuum-dried at 60°C for 18 hours, yielding 73 g of a particle powder (aggregated particle powder) composed of approximately spherical aggregated particles.

Then, 30 g of the above aggregated particle powder was immersed in a polymerizable monomer solution obtained by mixing 7 g of UDMA as a polymerizable monomer, 0.015 g of azobisisobutyronitrile (AIBN) as the thermal polymerization initiator, and 12.4 g of ethanol as an organic solvent. After thorough stirring to confirm that the mixture had become a slurry, it was allowed to stand for 1 hour. The mixture was then dried using a vacuum dryer under a reduced pressure of 10 hectopascals and a heating condition of 40°C for 1 hour to remove the organic solvent. After removing the organic solvent, a non-aggregating, highly fluid powder was obtained.

The above powder was heated under a reduced pressure of 10 hectopascals at 140°C for 20 minutes to polymerize and cure the polymerizable monomer in the powder. Then, the powder was sieved through a 100 $\mu$m mesh sieve, yielding 26 g of an organic-inorganic composite particle powder (D): FD-1, composed of substantially spherical organic-inorganic composite particles in which the surface of the obtained spherical aggregates was coated with an organic polymer.

An organic-inorganic composite particle powder: FD-2 was obtained in the same manner as FD-1, except that the amount of the polymerizable monomer UDMA used was changed to 4.1 g.

An organic-inorganic composite particle powder: FD-3 was obtained in the same manner as FD-1, except that the amount of the polymerizable monomer UDMA used was changed to 16 g.

An organic-inorganic composite particle powder: FD-4 was obtained in the same manner as FD-1, except that the amount of the polymerizable monomer UDMA used was changed to 1.6 g.

An organic-inorganic composite particle powder: FD-5 was obtained in the same manner as FD-1, except that the amount of the polymerizable monomer UDMA used was changed to 22.6 g.

The powder recovered from the sieve during the production of FD-1 was designated as an organic-inorganic composite particle powder: FD-6.

By pulverizing FD-6 using a vibrating ball mill, organic-inorganic composite particle powders with different average particle diameters: FD-7 to FD-9 were obtained.

The average particle diameter (median diameter in the volume-based particle size distribution) of the obtained organic-inorganic composite particle powder (D) was determined as follows. The results are shown in Table 4.

<Evaluation of average particle diameter of organic-inorganic composite particle powder>

0.1 g of the organic-inorganic composite particle powder (D) was dispersed in 10 mL of ethanol, and was irradiated with ultrasonic waves for 20 minutes. The average particle diameter was determined from the median diameter of the volume statistics by applying the optical model "Fraunhofer" using a laser diffraction-scattering particle size distribution analyzer "LS230" (manufactured by Beckman Coulter).

[Table 4]

| | Content ratio of inorganic particle powder (A) in organic-inorganic composite particles (d) constituting organic-inorganic composite particle powder (D) | | Average particle diameter ($\mu$m) |
|---|---|---|---|
| | Abbreviation | % by mass | |
| FD-1 | FA-2 | 81 | 45 |
| FD-2 | FA-2 | 88 | 64 |
| FD-3 | FA-2 | 65 | 42 |
| FD-4 | FA-2 | 95 | 65 |

(continued)

| | Content ratio of inorganic particle powder (A) in organic-inorganic composite particles (d) constituting organic-inorganic composite particle powder (D) | | Average particle diameter ($\mu$m) |
|---|---|---|---|
| | Abbreviation | % by mass | |
| FD-5 | FA-2 | 57 | 43 |
| FD-6 | FA-2 | 81 | 121 |
| FD-7 | FA-2 | 81 | 91 |
| FD-8 | FA-2 | 81 | 12 |
| FD-9 | FA-2 | 81 | 0.7 |

3. Examples and Comparative Examples

<Example 1>

A polymerizable monomer solution was prepared by completely dissolving 0.20 parts by mass of CQ and 0.5 parts by mass of DMBE as polymerization initiators in a polymerizable monomer composed of 60 parts by mass of UDMA and 40 parts by mass of 3G. Thereafter, 140 parts by mass of the inorganic particle powder (A) FA-2, 93 parts by mass of the inorganic particle powder (C) FC-4, and the polymerizable monomer solution were kneaded in a mortar until being homogeneous to form a paste, and then defoamed to prepare a paste-like dental curable composition. The prepared paste-like dental curable composition was filled into a cylindrical syringe, and a plunger for extruding the contents of the syringe and a cap were attached. One of the syringes filled with the prepared dental curable composition was then stored in a 50°C incubator for one week.

The obtained dental curable composition was evaluated for flowability in the paste state, consistency, dispersibility of crystalline rare earth metal fluoride in the curable composition, contrast ratio, flexural strength, and discharging feel using the methods described below.

The flowability was evaluated for the dental curable composition immediately after preparation and after storage at 50°C for one week. The evaluation result immediately after preparation was taken as an "initial" value (initial value), and the evaluation result after storage at 50°C for one week was taken as an "after storage" value (after-storage value). The results are shown in Table 5.

<Method for measuring flowability>

A 20G needle tip was attached to the tip of the syringe filled with the prepared paste-like dental curable composition. Then, after allowing the syringe to stand for 30 minutes in a constant temperature chamber at 25°C, a circle with a diameter of 5 mm was drawn on a glass plate in advance, 0.1 g of the dental curable composition was discharged into the circle, and the paste was allowed to stand in a horizontal state for 2 minutes in an incubator at 37°C. The vertical diameter and the horizontal diameter of the spread of the paste were measured, and the average of the vertical diameter and the horizontal diameter was calculated. The above evaluation was performed twice, and the average value was taken as the flowability of the paste. For fluidity, the degree of change due to storage was evaluated using the formula: {(after-storage value - initial value) / initial value} $\times$ 100 as the rate of change (%).

<Method for measuring consistency>

The prepared paste-like dental curable composition was allowed to stand in an incubator at 45°C for 1 day, and then the paste allowed to stand at 25°C for 30 minutes was measured for consistency by the following method. 0.2 g of the paste was weighed onto polypropylene film with a raised center. Polypropylene film, a glass plate, and a weight (50 g in total) were placed thereon in this order, and after 10 seconds had passed, the glass plate and the weight were removed, and the vertical diameter and the horizontal diameter of the paste were measured through the polypropylene film, and the average of the vertical diameter and the horizontal diameter was calculated. The above evaluation was performed twice, and the average value was taken as the consistency of the paste.

<Method for evaluating dispersibility of crystalline rare earth metal fluoride particles in dental curable composition>

The prepared dental curable composition was placed in a mold having a through hole of 7 mm$\varphi \times$ 1 mm, and a polyester film was pressed against both surfaces. Then, the polyester film was peeled off from only one side, and the peeled surface was irradiated with light with a dental light curing unit (Elipar DeepCure, manufactured by 3M) at a distance of 3 mm for 20 seconds. After curing the dental curable composition, it was taken out from the mold and placed in 10 mL of ethanol, then treated with ultrasound for 20 minutes. The resulting cured body was fixed to a sample stage with carbon tape, with the light-irradiated surface facing upwards, and a measurement sample was prepared by applying a conductive treatment (platinum vapor deposition). The measurement sample was observed using a scanning electron microscope ("JSM-7800F PRIME" manufactured by JEOL) at a magnification of 1,000x to obtain a backscattered electron image, and the number of particles having high brightness of 3 $\mu$m or larger observed within the unit field of view of the photograph was counted.

A: no particles having high brightness of 3 $\mu$m or larger are observed within the unit field of view of the scanning electron microscope

B: particles having high brightness of 3 $\mu$m or larger are observed within the unit field of view of the scanning electron microscope

<Method for evaluating contrast ratio (Yb/Yw) of cured product of dental curable composition>

The prepared dental curable composition was placed in a mold having a through hole of 7 mm$\varphi \times$ 1 mm, and polyester film was pressed against both surfaces. Then, the distance was adjusted such that the irradiation intensity on the surface of the polyester film was 1000 mW/cm$^2$, and both surfaces were irradiated with light for 20 seconds each using a dental light curing unit (Elipar DeepCure, manufactured by 3M). After curing the dental curable composition, it was taken out from the mold, and the Y values (background colors black and white) of the tristimulus values of the cured product were measured using a color difference meter ("TC-1800MKII" manufactured by Tokyo Denshoku Co., Ltd.). The contrast ratio (Yb/Yw) was calculated based on the following formula:

Contrast ratio (Yb/Yw) = (Y value when background color is black)/(Y value when background color is white)

<Method for measuring flexural strength>

The paste of the dental curable composition was filled in a stainless steel mold, and in a state of being pressed against polypropylene film, light irradiation was performed from one surface thereof three times for 30 seconds by using a visible light irradiator (Elipar DeepCure, manufactured by 3M) while bringing into close contact with the polypropylene film at different positions so that the entire surface was irradiated with light. Next, the opposite surface was also irradiated in the same manner with light three times for 30 seconds while bringing into close contact with the polypropylene film to obtain a cured product. The cured product was shaped into a prismatic shape of 2 $\times$ 2 $\times$ 25 mm using #1500 water resistant abrasive paper, and this sample piece was attached to a testing machine ("Autograph AG5000D" manufactured by Shimadzu Corporation). The three point bending fracture strength was measured at an inter-fulcrum distance of 20 mm and a crosshead speed of 1 mm/min, a load-deflection curve was obtained, and the flexural strength was obtained from the following formula. The evaluation was performed on five test pieces, and the average value was defined as the flexural strength.

$$\text{Formula: } \sigma B = (3PS) / (2WB^2)$$

The symbols in the above formula represent: $\sigma$B: flexural strength (Pa), P: load at the time of test piece fracture (N), S: inter-fulcrum distance (m), W: width of test piece (m), B: thickness of test piece (m), respectively.

<Evaluation of discharging feel>

A 20G needle tip was attached to the tip of the syringe container, and 0.2 g of paste was discharged from the tip of the needle tip onto a glass plate (5 cm $\times$ 10 cm) by pushing the plunger. The ease of pushing the plunger was checked, and the discharging feel of the paste was evaluated according to the following evaluation criteria. A rating of 1 to 3 of the discharging feel was considered acceptable.

1: Paste can be discharged with weak pressure, and discharging properties are very good.
2: Paste can be discharged without difficulty, and discharging properties are good.
3: Paste can be discharged with slightly stronger pressure, and discharging properties are acceptable.
4: Paste can be discharged with strong pressure, but discharging properties are poor.

5: Paste cannot be discharged at all.

<Examples 2 to 28, Comparative Examples 1 to 8>

A paste-like dental curable composition was prepared in the same manner as in Example 1, except that the polymerizable monomer (M), the inorganic particle powder (A), the inorganic particle powder (B), the inorganic particle powder (C), and the organic-inorganic composite particle powder (D) used were changed as shown in Tables 5 to 8, and the obtained composition was evaluated in the same manner as in Example 1. The results are shown in Tables 5, 6, 7, and 8.

[Table 5]

| | | | Ex. 1 | Ex. 2 | Ex. 3 | Ex. 4 | Ex. 5 | Ex. 6 | Ex. 7 | Ex. 8 | Ex. 9 | Ex. 10 |
|---|---|---|---|---|---|---|---|---|---|---|---|---|
| Polymerizable monomer (M) | UDMA | | 60 | 60 | 60 | 60 | 60 | 60 | 60 | 60 | 60 | 60 |
| | 3G | | 40 | 40 | 40 | 40 | 40 | 40 | 40 | 40 | 40 | 40 |
| Inorganic particle powder (A) | FA-1 | Parts by mass | | | | | | | 47 | | | |
| | FA-2 | | 140 | | 23 | 77 | 120 | 30 | | | | |
| | FA-3 | | | | | | | | | 47 | | |
| | FA-4 | | | | | | | | | | 47 | |
| | FA-5 | | | | | | | | | | | 47 |
| Inorganic particle powder (C) | FC-4 | | 93 | 95 | 70 | 16 | 15 | 90 | 47 | 47 | 47 | 47 |
| Organic-inorganic composite particle powder (D) | FD-1 | | | 176 | 140 | 140 | 165 | 180 | 140 | 140 | 140 | 140 |
| Inorganic particle powder (A) independent of the form of inclusion | | | 187 | 190 | 171 | 199 | 261 | 221 | 183 | 183 | 183 | 183 |
| Inorganic particle powder (B) independent of the form of inclusion | | | 46 | 47 | 35 | 8 | 8 | 45 | 23 | 23 | 23 | 23 |
| Flowability (mm) | Initial | | 4.1 | 4 | 4.2 | 5.2 | 4.3 | 5.5 | 4.2 | 4.0 | 4.2 | 4.1 |
| | After storage | | 4.2 | 4.1 | 4.4 | 5.6 | 4.5 | 6.0 | 4.3 | 4.0 | 4.4 | 4.2 |
| | Change rate (%) | | 2% | 2% | 5% | 8% | 5% | 9% | 2% | 0% | 5% | 2% |
| Consistency (mm) | | | 15 | 16 | 16 | 18 | 16 | 18.5 | 17 | 16 | 16 | 16 |
| Dispersibility | | | A | A | A | A | A | A | A | A | A | A |
| Contrast ratio (Yb/Yw) | | | 0.26 | 0.27 | 0.30 | 0.26 | 0.28 | 0.27 | 0.28 | 0.28 | 0.28 | 0.25 |
| Flexural strength (MPa) | | | 152 | 144 | 124 | 121 | 146 | 115 | 136 | 132 | 133 | 128 |
| Discharging feel | | | 3 | 2 | 1 | 1 | 2 | 1 | 1 | 1 | 1 | 1 |

[Table 6]

| | | | Ex. 11 | Ex. 12 | Ex. 13 | Ex. 14 | Ex. 15 | Ex. 16 | Ex. 17 | Ex. 18 | Ex. 19 | Ex. 20 |
|---|---|---|---|---|---|---|---|---|---|---|---|---|
| Polymerizable monomer (M) | UDMA | Parts by mass | 60 | 60 | 60 | 60 | 60 | 60 | 60 | 60 | 60 | 60 |
| | 3G | | 40 | 40 | 40 | 40 | 40 | 40 | 40 | 40 | 40 | 40 |
| Inorganic particle powder (A) | FA-2 | | 47 | 47 | 47 | 47 | 47 | 47 | 47 | 47 | 47 | 47 |
| Inorganic particle powder (C) | FC-1 | | 47 | | | | | | | | | |
| | FC-2 | | | 47 | | | | | | | | |
| | FC-3 | | | | 47 | | | | | | | |
| | FC-4 | | | | | | | | | | | |
| | FC-5 | | | | | | 47 | | | | | |
| | FC-6 | | | | | | | 47 | | | | |
| | FC-7 | | | | | | | | 47 | | | |
| | FC-8 | | | | | | | | | 47 | | |
| | FC-9 | | | | | | | | | | 47 | |
| | FC-10 | | | | | | | | | | | 47 | |
| | FC-11 | | | | | | | | | | | 47 |
| Organic-inorganic composite particle powder (D) | FD-1 | | 140 | 140 | 140 | 140 | 140 | 140 | 140 | 140 | 140 | 140 |
| Inorganic particle powder (A) independent of the form of inclusion | | | 183 | 183 | 183 | 183 | 183 | 183 | 183 | 183 | 183 | 183 |
| Inorganic particle powder (B) independent of the form of inclusion | | | 9 | 9 | 19 | 33 | 23 | 23 | 23 | 28 | 23 | 23 |
| Flowability (mm) | Initial | | 4.5 | 4.6 | 4.1 | 4.2 | 4.1 | 4.4 | 4.3 | 4 | 4 | 4.1 |
| | After storage | | 4.9 | 5.0 | 4.3 | 4.3 | 4.3 | 4.4 | 4.4 | 4.2 | 4.1 | 4.4 |
| | Change rate (%) | | 9% | 9% | 5% | 2% | 5% | 0% | 2% | 5% | 2% | 7% |
| Consistency (mm) | | | 17.4 | 17.5 | 16 | 16 | 16 | 16 | 16 | 16 | 16 | 16 |
| Dispersibility | | | A | A | A | A | A | A | A | A | A | A |
| Contrast ratio (Yb/Yw) | | | 0.27 | 0.27 | 0.28 | 0.28 | 0.47 | 0.28 | 0.28 | 0.28 | 0.28 | 0.27 |

EP 4 778 504 A1

23

(continued)

| | Ex. 11 | Ex. 12 | Ex. 13 | Ex. 14 | Ex. 15 | Ex. 16 | Ex. 17 | Ex. 18 | Ex. 19 | Ex. 20 |
|---|---|---|---|---|---|---|---|---|---|---|
| Flexural strength (MPa) | 130 | 135 | 141 | 141 | 129 | 143 | 131 | 142 | 132 | 139 |
| Discharging feel | 1 | 1 | 1 | 1 | 1 | 1 | 1 | 1 | 1 | 1 |

[Table 7]

| | | | Ex. 21 | Ex. 22 | Ex. 23 | Ex. 24 | Ex. 25 | Ex. 26 | Ex. 27 | Ex. 28 |
|---|---|---|---|---|---|---|---|---|---|---|
| Polymerizable monomer (M) | UDMA | Parts by mass | 60 | | | 60 | 60 | 60 | 60 | 60 |
| | 3G | | 40 | 40 | 40 | 40 | 40 | 40 | 40 | 40 |
| | GMA | | | 60 | | | | | | |
| | D-2.6E | | | | 60 | | | | | |
| Inorganic particle powder (A) | FA-2 | | 47 | 47 | 47 | 47 | 70 | 47 | 47 | 47 |
| Inorganic particle powder (B) | FB-2 | | | | | | | | | |
| Inorganic particle powder (C) | FC-4 | | | 47 | 47 | 47 | 23 | 47 | 47 | |
| | FC-12 | | 47 | | | | | | | |
| | FC-17 | | | | | | | | | 47 |
| Organic-inorganic composite particle powder (D) | FD-1 | | 140 | 140 | 140 | | | | | 140 |
| | FD-2 | | | | | 140 | | | | |
| | FD-3 | | | | | | 140 | | | |
| | FD-7 | | | | | | | 140 | | |
| | FD-8 | | | | | | | | 140 | |
| Inorganic particle powder (A) independent of the form of inclusion | | | 183 | 183 | 183 | 193 | 173 | 183 | 183 | 183 |
| Inorganic particle powder (B) independent of the form of inclusion | | | 23 | 23 | 23 | 23 | 12 | 23 | 23 | 23 |
| Flowability (mm) | Initial | | 4.2 | 4.2 | 4.1 | 4.6 | 4.2 | 4.4 | 4.1 | 4 |
| | After storage | | 4.3 | 4.3 | 4.1 | 5.0 | 4.4 | 4.5 | 4.2 | 4 |
| | Change rate (%) | | 2% | 2% | 0% | 9% | 5% | 2% | 2% | 0% |
| Consistency (mm) | | | 16 | 16 | 16 | 16 | 16 | 16 | 16 | 16 |
| Dispersibility | | | A | A | A | A | A | A | A | A |
| Contrast ratio (Yb/Yw) | | | 0.28 | 0.43 | 0.40 | 0.28 | 0.27 | 0.28 | 0.28 | 0.27 |
| Flexural strength (MPa) | | | 133 | 143 | 131 | 124 | 137 | 134 | 140 | 142 |
| Discharging feel | | | 1 | 1 | 1 | 1 | 1 | 1 | 1 | 1 |

[Table 8]

| | | | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 |
|---|---|---|---|---|---|---|---|---|---|---|
| Polymerizable monomer (M) | UDMA | | 60 | 60 | 60 | 60 | 60 | 60 | 60 | 60 |
| | 3G | | 40 | 40 | 40 | 40 | 40 | 40 | 40 | 40 |
| Inorganic particle powder (A) | FA-2 | | 15 | 67 | 70 | | 30 | | 85 | 82 |
| Inorganic particle powder (B) | FB-2 | | | | | | | | | 12 |

(continued)

| | | | Comp. Ex. 1 | Comp. Ex. 2 | Comp. Ex. 3 | Comp. Ex. 4 | Comp. Ex. 5 | Comp. Ex. 6 | Comp. Ex. 7 | Comp. Ex. 8 |
|---|---|---|---|---|---|---|---|---|---|---|
| Inorganic particle powder (C) | FC-4 | Parts by mass | 105 | 7 | | 95 | 90 | 95 | 16 | |
| | FC-13 | | | | 12 | | | | | |
| Organic-inorganic composite particle powder (D) | FD-1 | | 180 | 111 | 152 | | | | | 140 |
| | FD-4 | | | | | 176 | | | | |
| | FD-5 | | | | | | 180 | | | |
| | FD-6 | | | | | | | 176 | | |
| | FD-9 | | | | | | | | 102 | |
| Inorganic particle powder (A) independent of the form of inclusion | | | 213 | 161 | 199 | 214 | 178 | 190 | 176 | 201 |
| Inorganic particle powder (B) independent of the form of inclusion | | | 53 | 4 | 9 | 47 | 45 | 47 | 8 | 12 |
| Flowability (mm) | Initial | | 4.1 | 6.3 | 4.4 | 4.4 | 4.3 | 4.2 | 4.1 | 4.5 |
| | After storage | | 4.2 | 7.1 | 5.1 | 4.7 | 4.5 | 4.5 | 4.2 | 5.8 |
| | Change rate (%) | | 2% | 13% | 16% | 7% | 5% | 7% | 2% | 29% |
| Consistency (mm) | | | 16 | 19 | 18 | 16 | 16 | 16 | 16 | 18 |
| Dispersibility | | | A | A | B | A | A | A | A | B |
| Contrast ratio (Yb/Yw) | | | 0.30 | 0.26 | 0.27 | 0.28 | 0.28 | 0.28 | 0.28 | 0.28 |
| Flexural strength (MPa) | | | 96 | 132 | 137 | 105 | 135 | 95 | 141 | 133 |
| Discharging feel | | | 4 | 1 | 2 | 1 | 4 | 2 | 4 | 1 |

As can be understood from the results of Examples 1 to 28, when using inorganic particle powder and organic-inorganic composite particle powder that meet the requirements of the present invention, the crystalline rare earth metal fluoride is sufficiently dispersed in the curable composition, and the change in fluidity over time is small. Fig. 2 shows a photograph of the cured product of the dental curable composition of Example 1 observed with a scanning electron microscope, and no particles with high brightness of 3 $\mu$m or larger were observed, indicating that the dispersibility of the crystalline rare earth metal fluoride is good.

As can be understood from the results of Comparative Examples 1 and 2, when the amount of the inorganic particle powder (B), regardless of its form of inclusion, does not meet the requirements of the present invention, a decrease in strength and deterioration of discharging feel, or a significant change in fluidity over time due to storage, is observed.

As can be understood from the results of Comparative Example 3, when the inorganic particle powder (C) does not meet the requirements of the present invention, the crystalline rare earth fluoride particles are not sufficiently dispersed in the curable composition, and a significant change in fluidity over time due to storage is observed.

As can be understood from the results of Comparative Examples 4 to 7, when the organic-inorganic composite particle powder (D) do not meet the requirements of the present invention, the strength of the cured product of the dental curable composition decreases or the discharging feel deteriorates.

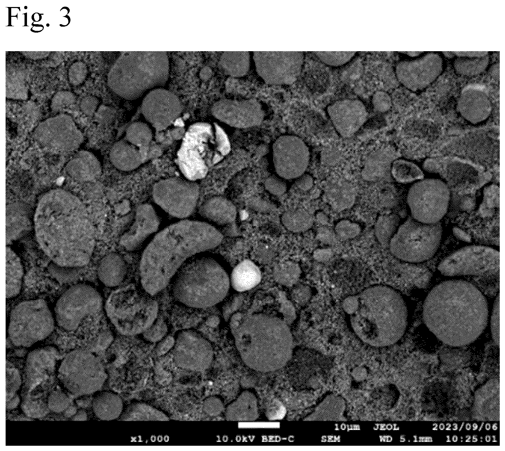

Fig. 3 shows a photograph of the cured product of the dental curable composition of Comparative Example 8 observed with a scanning electron microscope, and particles with high brightness of 3 $\mu$m or larger are observed, indicating poor dispersibility of the crystalline rare earth metal fluoride. As can be understood from the result, when the inorganic particle powder (B) is added directly, aggregated particles remain in the curable composition and are not sufficiently dispersed, resulting in a large change in fluidity over time.

**Claims**

1. A dental curable composition comprising a composition containing

100 parts by mass of a polymerizable monomer (M), and
10 to 100 parts by mass of an inorganic particle powder (C),
the inorganic particle powder (C) composed of aggregated particles (c) consisting of inorganic particles (a) that constitute an inorganic particle powder (A) having an average primary particle diameter of 50 nm to 1 $\mu$m as measured by electron microscopy, the inorganic particle powder (A) composed of a plurality of inorganic particles (a) composed of the same type of material, exhibiting a negative zeta potential polarity when measured in water, and inorganic particles (b) that constitute an inorganic particle powder (B) having an average primary particle diameter of 1 to 300 nm as measured by electron microscopy, the inorganic particle powder (B) composed of a plurality of inorganic particles (b) composed of the same type of material, exhibiting a positive zeta potential polarity when measured in water,
an average value of a total mass of the inorganic particles (b) relative to a total mass of 100 parts by mass of the inorganic particles (a) contained in each of the aggregated particles (c) that constitute the inorganic particle powder (C) being within a range of 20 to 300 parts by mass,
the inorganic particle powder (C) having an average aggregate particle diameter of 1 to 50 $\mu$m, defined as a median diameter in a volume-based particle size distribution measured by a laser diffraction-scattering method,
wherein the dental curable composition further contains at least one of the inorganic particle powder (A) and an organic-inorganic composite particle powder (D),
the organic-inorganic composite particle powder (D) is composed of organic-inorganic composite particles (d) consisting of a composite material, which is a composite material of the inorganic particle powder (A) and a resin, and in which the inorganic particle powder (A) has a content of 60 to 90% by mass in the composite material, and
the organic-inorganic composite particle powder (D) has an average particle diameter of 1 to 100 $\mu$m, defined as a median diameter in the volume-based particle size distribution measured by a laser diffraction-scattering method,
the inorganic particle powder (A) has a total content of, regardless of its form of inclusion, 170 to 270 parts by mass, and the inorganic particle powder (B) has a total content of, regardless of its form of inclusion, 5 to 50 parts by mass in the composition.

2. The dental curable composition according to claim 1, wherein, in each aggregated particle (c) constituting the inorganic particle powder (C), the inorganic particles (b) are uniformly dispersed in the inorganic particles (a).

3. The dental curable composition according to claim 1, wherein the inorganic particles (a) consist of a silica-based inorganic compound, and the inorganic particles (b) consist of a crystalline rare earth metal fluoride.

4. The dental curable composition according to claim 3, wherein a full width at half maximum of a maximum intensity peak derived from the crystalline rare earth metal fluoride in an X-ray diffraction pattern obtained when X-ray diffraction measurement is performed on the inorganic particle powder (B) is 0.3° or more.

5. The dental curable composition according to claim 1, wherein the inorganic particle powder (C) is obtained by spray drying a homogeneous mixed slurry obtained by mixing a slurry (Sa) in which 100 parts by mass of the inorganic particle powder (A) are dispersed in a dispersion medium, and a slurry (Sb) in which 20 to 300 parts by mass of the inorganic particle powder (B) are dispersed in a dispersion medium.

6. A method for producing the dental curable composition according to claim 1, comprising mixing:

the polymerizable monomer (M),
the inorganic particle powder (C), and
at least one of the inorganic particle powder (A) and the organic-inorganic composite particle powder (D).

7. The method for producing the dental curable composition according to claim 6, wherein the inorganic particle powder (C) is produced by spray drying a homogeneous mixed slurry obtained by mixing a slurry (Sa) in which 100 parts by mass of the inorganic particle powder (A) are dispersed in a dispersion medium, and a slurry (Sb) in which 20 to 300 parts by mass of the inorganic particle powder (B) are dispersed in a dispersion medium.

8. The method for producing the dental curable composition according to claim 7, wherein the mixed slurry does not contain an ionic surfactant.

9. The method for producing the dental curable composition according to claim 7, wherein the mixed slurry has a concentration of less than 40% by mass.

10. An inorganic particle powder, which is an inorganic particle powder (C) composed of aggregated particles (c) consisting of inorganic particles (a) that constitute an inorganic particle powder (A) having an average primary particle diameter of 50 nm to 1 $\mu$m as measured by electron microscopy, the inorganic particle powder (A) composed of a plurality of inorganic particles (a) composed of the same type of material, exhibiting a negative zeta potential polarity when measured in water, and inorganic particles (b) that constitute an inorganic particle powder (B) having an average primary particle diameter of 1 to 300 nm as measured by electron microscopy, the inorganic particle powder (B) composed of a plurality of inorganic particles (b) composed of the same type of material, exhibiting a positive zeta potential polarity when measured in water,

an average value of a total mass of the inorganic particles (b) relative to a total mass of 100 parts by mass of the inorganic particles (a) contained in each of the aggregated particles (c) that constitute the inorganic particle powder (C) being within a range of 20 to 300 parts by mass,
the inorganic particle powder having an average aggregate particle diameter of 1 to 50 $\mu$m, defined as a median diameter in a volume-based particle size distribution measured by a laser diffraction-scattering method, and being free of an ionic surfactant.

11. A method for producing the inorganic particle powder according to claim 10, including a step of spray drying a homogeneous mixed slurry obtained by mixing a slurry (Sa) in which 100 parts by mass of the inorganic particle powder (A) are dispersed in a dispersion medium, and a slurry (Sb) in which 20 to 300 parts by mass of the inorganic particle powder (B) are dispersed in a dispersion medium, wherein the mixed slurry does not contain an ionic surfactant.

12. The method for producing the inorganic particle powder according to claim 11, wherein the mixed slurry has a concentration of less than 40% by mass.

Fig. 1

Fig. 2

Fig. 3

## INTERNATIONAL SEARCH REPORT

International application No.

**PCT/JP2024/038942**

| A. | CLASSIFICATION OF SUBJECT MATTER |
|---|---|

*A61K 6/30*(2020.01)i; *A61K 6/15*(2020.01)i; *A61K 6/17*(2020.01)i; *A61K 6/70*(2020.01)i; *A61K 6/831*(2020.01)i; *A61K 6/842*(2020.01)i; *A61K 6/878*(2020.01)i; *C01B 33/12*(2006.01)i

FI: A61K6/30; A61K6/70; A61K6/15; A61K6/831; A61K6/842; A61K6/17; C01B33/12 A; A61K6/878

According to International Patent Classification (IPC) or to both national classification and IPC

| B. | FIELDS SEARCHED |
|---|---|

Minimum documentation searched (classification system followed by classification symbols)

A61K6/30; A61K6/15; A61K6/17; A61K6/70; A61K6/831; A61K6/842; A61K6/878; C01B33/12

Documentation searched other than minimum documentation to the extent that such documents are included in the fields searched

Published examined utility model applications of Japan 1922-1996
Published unexamined utility model applications of Japan 1971-2025
Registered utility model specifications of Japan 1996-2025
Published registered utility model applications of Japan 1994-2025

Electronic data base consulted during the international search (name of data base and, where practicable, search terms used)

JSTPlus/JMEDPlus/JST7580 (JDreamIII); CAplus/MEDLINE/EMBASE/BIOSIS (STN)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | WO 2013/039169 A1 (TOKUYAMA DENTAL CORP.) 21 March 2013 (2013-03-21) paragraphs [0081], [0184]-[0194], [0205] | 1-12 |
| A | JP 2014-177443 A (TOKUYAMA DENTAL CORP.) 25 September 2014 (2014-09-25) claims 1-16, paragraphs [0016], [0018]-[0019], [0037], [0042], [0096], [0175]-[0176], [0179], [0205] | 1-12 |
| A | WO 2023/085201 A1 (TOKUYAMA DENTAL CORP.) 19 May 2023 (2023-05-19) claims 1-5, paragraphs [0033]-[0035], [0067]-[0070], [0076], [0079]-[0080], [0083], [0092]-[0095] | 1-12 |

☐ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "D" document cited by the applicant in the international application | "X" document of particular relevance; the claimed invention cannot be considered novel or cannot be considered to involve an inventive step when the document is taken alone |
| "E" earlier application or patent but published on or after the international filing date | |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | "&" document member of the same patent family |
| "P" document published prior to the international filing date but later than the priority date claimed | |

| Date of the actual completion of the international search | Date of mailing of the international search report |
|---|---|
| **09 January 2025** | **21 January 2025** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (July 2022)

**INTERNATIONAL SEARCH REPORT**
**Information on patent family members**

International application No.

**PCT/JP2024/038942**

| Patent document cited in search report | | | Publication date (day/month/year) | Patent family member(s) | Publication date (day/month/year) |
|---|---|---|---|---|---|
| WO | 2013/039169 | A1 | 21 March 2013 | US 2014/0213687 A1 paragraphs [0088], [0241]-[0252], [0260]<br>EP 2757112 A1 | |
| JP | 2014-177443 | A | 25 September 2014 | (Family: none) | |
| WO | 2023/085201 | A1 | 19 May 2023 | EP 4434512 A1 claims 1-5, paragraphs [0033]-[0035], [0067]-[0070], [0076], [0079]-[0080], [0083], [0092]-[0095] | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2023085201 A